# EUROPEAN PATENT SPECIFICATION

(11) **EP 3 341 485 B1**
(45) Date of publication and mention of the grant of the patent: **26.11.2025**
(21) Application number: 16840048.9
(22) Date of filing: 24.08.2016
(51) Int. Cl.: A01K 67/027, A61K 39/395, C07K 16/00, C12N 15/13

(54) **ENHANCED PRODUCTION OF IMMUNOGLOBULINS**
ERHÖHTE PRODUKTION VON IMMUNGLOBULINEN
PRODUCTION AMÉLIORÉE D'IMMUNOGLOBULINES

(30) Priority: 24.08.2015 US 201562209267 P
(43) Date of publication of application: 04.07.2018
(73) Proprietor: Trianni, Inc., San Francisco, CA 94122 (US)
(72) Inventor: WABL, Matthias, San Francisco, CA 94122 (US); DUONG, Bao, San Francisco, CA 94122 (US)
(74) Representative: Redl, Gerda
(86) International application number: PCT/US2016/048436
(87) International publication number: WO 2017/035252

(56) References cited:
- WO-A1-2012/123949
- WO-A1-2013/022782
- WO-A1-2013/138681
- WO-A2-2009/157771
- WO-A2-2013/171505
- US-A- 4 892 824
- US-A1- 2012 090 041
- RAFAEL CASELLAS ET AL: "Ig[kappa] allelic inclusion is a consequence of receptor editing", JOURNAL OF EXPERIMENTAL MEDICINE, vol. 204, no. 1, 22 January 2007 (2007-01-22), US, pages 153 - 160, XP055536933, ISSN: 0022-1007, DOI: 10.1084/jem.20061918
- EIICHIRO SONODA ET AL: "B Cell Development under the Condition of Allelic Inclusion", IMMUNITY., vol. 6, no. 3, 1 March 1997 (1997-03-01), US, pages 225 - 233, XP055536929, ISSN: 1074-7613, DOI: 10.1016/S1074-7613(00)80325-8
- CHRISTIAN VETTERMANN ET AL: "Allelic exclusion of immunoglobulin genes: models and mechanisms : Allelic exclusion of immunoglobulin genes", IMMUNOLOGICAL REVIEWS., vol. 237, no. 1, 19 August 2010 (2010-08-19), US, pages 22 - 42, XP055536935, ISSN: 0105-2896, DOI: 10.1111/j.1600-065X.2010.00935.x
- KANNAN GUNASEKARAN ET AL: "Enhancing Antibody Fc Heterodimer Formation through Electrostatic Steering Effects : APPLICATIONS TO BISPECIFIC MOLECULES AND MONOVALENT IgG", JOURNAL OF BIOLOGICAL CHEMISTRY, vol. 285, no. 25, 16 April 2010 (2010-04-16), US, pages 19637 - 19646, XP055546816, ISSN: 0021-9258, DOI: 10.1074/jbc.M110.117382
- DATABASE UniProt [online] 4 February 2015 (2015-02-04), "SubName: Full=Ig gamma-1 chain C region secreted form {ECO:0000313|Ensembl:ENSMUSP00000141658}; Flags: Fragment;", XP002788301, retrieved from EBI accession no. UNIPROT:A0A0A6YWR2 Database accession no. A0A0A6YWR2
- ZHI LIU ET AL: "A Novel Antibody Engineering Strategy for Making Monovalent Bispecific Heterodimeric IgG Antibodies by Electrostatic Steering Mechanism", JOURNAL OF BIOLOGICAL CHEMISTRY, vol. 290, no. 12, 20 March 2015 (2015-03-20), pages 7535 - 7562, XP055182700, ISSN: 0021-9258, DOI: 10.1074/jbc.M114.620260
- K RAJEWSKY ET AL: "Allelic exclusion model questioned", NATURE, vol. 359, 1 January 1992 (1992-01-01), pages 371 - 372, XP055547316
- WABL M ET AL: "Allelic exclusion model questioned.", NATURE 01 OCT 1992, vol. 359, no. 6394, 1 October 1992 (1992-10-01), pages 370, XP009510728, ISSN: 0028-0836
- KITAMURA, D ET AL.: "Targeted disruption of µ Chain Membrane Exon Causes Loss of Heavy-Chain Allelic Exclusion.", NATURE, vol. 356, 12 March 1992 (1992-03-12), pages 154 - 156, XP055366976
- CASELLAS, R ET AL.: "Igk Allelic Inclusion is a Consequence of Receptor Editing.", JOURNAL OF EXPERIMENTAL MEDICINE, vol. 204, no. 1, 22 January 2007 (2007-01-22), pages 153 - 160, XP055355291
- LUTZ, J ET AL.: "Pro-B Cells Sense Productive Immunoglobulin Heavy Chain Rearrangement Irrespective of Polypeptide Production.", PNAS., vol. 108, no. 26, 28 June 2011 (2011-06-28), pages 10644 - 10649, XP055365448

## Description

### FIELD OF THE INVENTION

This invention relates to the production of immunoglobulin molecules, including the production of bispecific antibodies in transgenic animals for the development of human therapeutics.

### BACKGROUND OF THE INVENTION

In the following discussion certain articles and methods are described for background and introductory purposes. Nothing contained herein is to be construed as an "admission" of prior art. Applicant expressly reserves the right to demonstrate, where appropriate, that the articles and methods referenced herein do not constitute prior art under the applicable statutory provisions.

Monoclonal antibodies have emerged as an important class of therapeutic molecules for the treatment of human diseases of various etiologies. In humans as well as most vertebrate animals, antibodies exist as dimers of two identical heavy (H) chains that are each paired with an identical light (L) chain. The N-terminus of each H and L chain consists of a variable domain (V_{H} and V_{L}, respectively) that together provides the H-L pair with its unique antigen-binding specificity. Thus, each antibody consists of two identical antigen-binding sites and is monospecific.

The exons that encode the antibody V_{H} and V_{L} domains do not exist in the germline DNA. Instead, each V_{H} exon is generated by the recombination of randomly selected V, D, and J genes present in the H chain locus; likewise, individual V_{L} exons are produced by the chromosomal rearrangements of randomly selected V and J genes in the light chain locus. The human genome contains two alleles that can express the H chain (one allele from each parent), two alleles that can express the kappa (κ) L chain, and two alleles that can express the lambda (λ) L chain. There are multiple V, D, and J genes at the H chain locus as well as multiple V and J genes at both L chain loci. Downstream of the J genes at each immunoglobulin locus exists one or more exons that encode the constant region of the antibody. In the heavy chain locus, exons for the expression of different antibody classes (isotypes) also exist. Despite the presence of multiple immunoglobulin alleles in the genome, each B cell is prevented from expressing more than one functional heavy chain and one functional light chain at a time by a process called allelic exclusion.

During B cell development, V(D)J gene recombination occurs first on one of the two homologous chromosomes that contain the H chain genes. The resultant V_{H} exon is subsequently spliced at the RNA level to the exons that encode the constant regions of the H chain (C_{H}). A full-length H chain can now be expressed only if the V_{H} exon formed following VDJ gene rearrangement is in-frame with the C_{H} exons. Once the H chain polypeptides are translated in the endoplasmic reticulum (ER), they form membrane-bound homodimers and pair with the VpreB and λ5 proteins to form the pre-B cell receptor (pre-BCR) complex. VpreB and λ5 together act as surrogate L chains, and only properly folded pre-BCRs can traffic to the cell surface from the ER. Once a sufficient number of pre-BCRs reach the cell surface, by mechanisms that are still incompletely understood, a signaling cascade is triggered to prevent enzymes of the recombinase activating genes (RAGs) from proceeding to recombine the V, D, and J genes of the second heavy chain allele on the homologous chromosome. By contrast, if a non-functional heavy chain gene is generated from the first heavy chain allele, no pre-BCRs are formed and the second H chain allele is now permissive for VDJ recombination. Thus, H chain allelic exclusion is mediated by signaling from the cell surface pre-BCRs. This is best exemplified by B cells in which the loss of Cµ heavy chain trasmembrane exons results in severely compromised heavy chain allelic exclusion (Kitamura and Rajewsky, Nature 356:154-156 (1992)).

Upon successful completion of the VDJ gene rearrangements for the production of a functional H chain, VJ recombination occurs at one of the L chain loci in a similar orderly fashion. In both humans and mice, the κ L chain locus tends to rearrange before the λ L chain locus. The VJ rearrangements occur on one L chain allele at a time until a functional L chain is produced, after which the L chain polypeptides can now associate with the H chain homodimers. As in the case of the pre-BCR assembly, only a functional B cell receptor (BCR) consisting of homodimeric H chains that are each paired to an L chain can traffic to the plasma membrane to mediate the signals necessary for further B cell development. The cell surface BCR signals also effectively turn off *RAG* expression to prevent any further L chain rearrangements. Thus, heavy chain expression on the plasma membrane is required to mediate allelic exclusion at both the H and L chain loci.

Because of allelic exclusion at the H and L chain loci, each B lymphocyte is capable of producing only monospecific antibodies. Therapeutically, however, artificially engineered antibodies that harbor two different antigen-binding sites per antibody molecule have been proven to be efficacious as treatments for a number of diseases. The generation of such bispecific antibodies typically involves time-consuming separate efforts to screen, identify, and isolate the monospecific antibodies against each the two distinct antigens of interest. Subsequently, the genes encoding the H and L chains of each candidate monoclonal antibody to be engineered as one half of a bispecific antibody are cloned and modified for permissive heterotypic associations between H chains or between H and L chains. To obtain bispecific antibodies for further evaluation, a cell line must be transfected with the modified H and/or L chain genes from the two original monoclonal antibodies. Thus, a method for more efficient production of bispecific antibodies, particularly during the initial phases of drug development, is an important unmet need. The methods and compositions provided by the present specification meet this important need.

WO2013171505A2 discloses a concept of guided selection of antibody variable domains, combination and expression entirely in vivo.

WO2013022782A1 provides mice, tissue, cells and genetic material that comprise a humanized heavy chain immunoglobulin locus, a humanized light chain locus that expresses a universal light chain, and a gene encoding an ADAM6 or ortholog or homolog or functional fragment thereof. Methods and compositions for making bispecific binding proteins are also disclosed.

Eiichiro Sonosa et al., Immunity, 1997, vol. 6, no. 3, pages 225-233, disclose mice whose IgH alleles are engineered to encode two distinct antibody heavy chains generate a normal-sized B cell compartment in which most cells stably express the two heavy chains, demonstrating that "toxicity" of bi-allelic exclusion H chain expression and cell autonomous mechanisms of silencing in-frame IgG gene rearrangements do not significantly contribute to allelic exclusion at the IgH locus.

Christian Vettermann et al., Immunological reviews, 2010, vol. 237, n. 1, pages 22-42, disclose models and mechanisms of allelic exclusion of immunoglobulin genes. They review the theoretical models that have been proposed to explain the establishment of Ig allelic exclusion and focus on the molecular mechanisms utilized by developing B cells to ensure the monoallelic expression of Igκ and Igλ light chain genes.

Kannan Gunasekaran et al., Journal of Biological Chemistry, 2010, vol. 285, no. 25, pages 19637-19646, disclose methods for enhancing antibody Fc heterodimer formation through electrostatic steering effects and applications to bispecific molecules and monovalent IgG.

UniProt database accession no. A0A0A6YWR2 discloses the sequence of the Immunoglobulin gamma-1 chain C region secreted form.

Zhi Liu et al., Journal of Biological Chemistry, 2015, vol. 290, no. 12, pages 7535-7562, disclose an antibody engineering strategy for making monovalent bispecific heterodimeric IgG antibodies by electrostatic steering mechanism.

K Rajewsky et al., Nature, 1992, vol. 359, pages 371-372, and Wabl M et al., Nature, 1992, vol 359, page 370, comment on an allelic exclusion model.

US2012/090041A1 discloses compositions and methods for inhibiting endogenous immunoglobulin genes and producing transgenic human idiotype antibodies. Disclosed are transgenic animals lacking endogenous Ig and capable of producing transgenic antibodies, as well as methods of making the same.

WO2012/123949A1 discloses bi- and monospecific, asymmetric antibodies and methods of generating the same.

Kitamura D. et al., Nature, 1992, vol 356, pages 154-156 disclose that targeted disruption of the membrane exon of the µ chain membrane exon causes loss of heavy-chain allelic exclusion.

Casellas R. et al., Journal of Experimental Medicine, 2007, vol. 204, no. 1, pages 153-160, disclose that Igκ allelic inclusion is a consequence of receptor editing.

Lutz J. et al., PNAS, 2011, vol. 108, no. 26, pages: 10644-10649, disclose that Pro-B cells sense productive immunoglobulin heavy chain rearrangement irrespective of polypeptide production.

WO2013138681A1 discloses mice that produce antigen-binding proteins with pH-dependent binding characteristics.

US4892824A discloses a method for producing monoclonal bi-specific immunoglobulins.

WO2009157771A2 discloses transgenic, non-human animals comprising a nucleic acid encoding an immunoglobulin light chain, whereby the immunoglobulin light chain is human, human-like, or humanized.

Aslam et al. (PNAS 117(49):31343-31352, 2020) disclose that Ig heavy chain protein but not its message controls early B cell development.

### SUMMARY OF THE INVENTION

This Summary is provided to introduce a selection of concepts in a simplified form that are further described below in the Detailed Description. This Summary is not intended to identify key or essential features of the claimed subject matter, nor is it intended to be used to limit the scope of the claimed subject matter. Other features, details, utilities, and advantages of the claimed subject matter will be apparent from the following written Detailed Description, including those aspects illustrated in the accompanying drawings and defined in the appended claims.

The present specification specifically describes the subject matter of the claims.:

According to a specific aspect, the invention provides a genetically modified rodent that produces B lymphocytes each capable of expressing a bispecific antibody, the genetically modified rodent comprising heavy chain alleles comprising unrearranged V, D and J genes, which alleles are engineered to introduce mutations that disfavor homodimeric heavy chain formation but are compatible with heterodimerization between the heavy chains expressed from the alleles, which mutations are any of:
(i) a mutation in an exon that encodes a murine IgG1 CH3 dimerization domain comprising D276K, E233K, and Q234K in a first heavy chain allele and K286D, K269D, and T247D in a second heavy chain allele, wherein amino acid numbering starts at the first codon of CH1; or
(ii) the heavy chains from each allele are fused to a protein or protein domain which is a complementary half of a heterodimerizer pair such as c-Fos and c-Jun, or to a leucine zipper that forms heterodimers.
According to a specific aspect, part or all of one or more constant region exons of an immunoglobulin heavy chain gene are in inverted reading frame orientation relative to rearranged V(D)J gene segments in the same immunoglobulin heavy chain gene, wherein the inverted constant region exons are immediately flanked by two oppositely oriented recognition sequences for site-specific DNA recombination.

According to a specific aspect, the genetically modified rodent further comprises a DNA cassette comprising one or more of: a splice acceptor, a ribosomal skip sequence, an internal ribosomal entry site (IRES), an open reading frame, a poly-adenylation signal sequence, and a stop codon inserted downstream of J gene segments of one allele in the heavy chain locus to prevent expression of the constant region (CH) exons downstream of the rearranged V(D)J gene segments on the same chromosome, wherein the DNA cassette is flanked by oppositely oriented site-specific recombinase recognition sequences such that the DNA cassette can be excised or inverted to allow a previously silenced VDJ exon to be spliced to a downstream CH exon for full-length heavy chain expression.

According to a specific aspect, the genetically modified rodent comprises an immunoglobulin heavy chain locus comprising mutant Cγ1 shown in SEQ ID NO: 1 and 2.

According to a specific aspect, the genetically modified rodent is a rat or a mouse.

According to a specific aspect, the invention further provides a B cell from the genetically modified rodent is capable of expressing an immunoglobulin comprising two functional V_{H} domains and one functional V_{L} domain, the B cell comprising a heavy chain encoding gene which is engineered in both heavy chain alleles to introduce mutations that disfavor homodimeric heavy chain formation but are compatible with heterodimerization between heavy chains expressed from the alleles, which mutations are any of:
(i) a mutation in an exon that encodes a murine IgG1 CH3 dimerization domain comprising D276K, E233K. and Q234K in a first heavy chain allele and K286D, K269D, and T247D in a second heavy chain allele, wherein amino acid numbering starts at the first codon of CH1; or
(ii) the heavy chains from each allele are fused to a protein or protein domain which is a complementary half of a heterodimerizer pair such as c-Fos and c-Jun, or to a leucine zipper that forms heterodimers.

According to a specific aspect, the invention further provides a method of producing a bispecific antibody, the method comprising:
(a) providing the genetically modified rodent described herein; and
(b) immunizing the genetically modified rodent with two different antigens simultaneously, or with one antigen followed by a second, different antigen, to generate B cells, each capable of co-expressing, or sequentially expressing, two different antigen receptors and/or a bispecific antigen receptor.

According to a specific aspect, the invention further provides a method of generating an immortalized B cell or hybridoma that expresses a bispecific antibody, the method comprising:
(a) providing the genetically modified rodent described herein;
(b) immunizing the genetically modified rodent with two different antigens simultaneously, or with one antigen followed by second, different antigen, to generate B cells, each capable of co-expressing, or sequentially expressing, two or more different antigen receptors and/or a bispecific antigen receptor;
(c) isolating B cells specific for both the first and second antigen; and
(d) generating immortalized B cells or a hybridoma using the isolated B cells.

According to a specific aspect, the bispecific antibody consists of heavy chains only.

According to a specific aspect, the bispecific antibody comprises two different light chain proteins and one heavy chain protein.

According to a specific aspect, the bispecific antibody comprises one light chain protein and two different heavy chain proteins.

According to a specific aspect, the invention further provides a method of cloning immunoglobulin chain genes, the method comprising:
(a) providing the genetically modified rodent described herein;
(b) immunizing the genetically modified rodent with two different antigens simultaneously, or with one antigen followed by second, different antigen, to activate B cells, each capable of co-expressing, or sequentially expressing, two different antigen receptors and/or a bispecific antigen receptor;
(c) isolating B cells specific for both the first and second antigen; and
(d) obtaining RNA from the isolated B cells to clone immunoglobulin heavy and light chain genes.

Specifically described herein are processes for the isolation of bispecific antibodies. It also describes transgenic animals, including transgenic mammals, carrying modified immunoglobulin alleles or other transgenes in their genomes. The modifications to the alleles interfere with the normal mechanisms of allelic exclusion following VDJ and/or VJ rearrangements. B cells in these genetically modified animals acquire the ability to produce antibodies with two or more antigen-binding specificities. Suppressing allelic exclusion to facilitate the production of bispecific antibodies from an immunized host is a specific aspect described herein.

In some specific aspects , the invention introduces modifications to the immunoglobulin alleles to impinge on the normal allelic exclusion processes such that an in-frame V(D)J rearrangement does not prevent the successive V(D)J rearrangement of the other immunoglobulin allele(s). By interfering with the signals that mediate allelic exclusion during B cell development, herein described are methods and compositions for modifying the genomic contents of animals so that their B cells are capable of expressing more than one functional V_{H} domain and/or or more than one functional V_{L} domain per cell.

According to a specific aspect, pre-BCR signaling is impaired by mutations introduced into each of the two H chain alleles to suppress heavy chain allelic exclusion. The mutations are also selected such that they disfavor homodimeric heavy chain formation but are compatible with heterodimerization between the two mutant heavy chains. As a consequence, heavy chain polypeptides expressed from either one of the two mutant heavy chain alleles alone are expected to misfold in the ER and thus be prevented from trafficking to the plasma membrane to mediate signals for allelic exclusion and further B cell development. Thus, successful pre-BCR assembly occurs only when both mutant heavy chain alleles are co-expressed and form complementary heterodimers. The constant regions of the heavy chain locus may also be modified to limit the extent of isotype switching. Although the individual B cells are expected to express two different heavy chains paired to only one kind of light chain, both the heavy and light chain repertoires are expected to be very extensive in the pool of B cells in a host.

In another specific aspect, the constant regions of the heavy chain and/or light chain alleles are modified such that an in-frame VDJ or VJ rearrangement on one allele is incapacitated for allelic exclusion but preserved for expression at a later B cell developmental stage. In one specific example, a DNA cassette is inserted downstream of the J genes of one allele in the heavy chain locus to prevent the expression of full-length heavy chains from a productively assembled VDJ exon. Such a DNA cassette may include one or more of the following elements: a splice acceptor, a ribosomal skip sequence or internal ribosomal entry site (IRES), an open reading frame, a poly-adenylation signal sequence, or a stop codon. Thus, an in-frame VDJ exon from this allele cannot mediate allelic exclusion or support further B cell development. However, B cells can still develop normally if an in-frame VDJ gene rearrangement occurs on the second allele. For subsequent reactivation of the silenced heavy chain allele, recognition sequences for a site-specific DNA recombinase, such as Cre, may be introduced to flank the inserted DNA cassette. When the site-specific DNA recombinase is expressed, the DNA cassette is now excised or inverted, allowing the previously silenced VDJ exon to be spliced to the other C_{H} exons downstream for full-length heavy chain expression. Alternatively, the DNA cassette may be inserted at such position that it can be excised from the heavy chain locus via normal isotype switching mechanisms. The downstream constant regions of both heavy chain alleles may be further modified to limit the extent of isotype switching, to favor heterodimerization when both heavy chains are co-expressed, or to allow only one heavy chain to be expressed at a time.

In another specific aspect, an analogous strategy to the one just described may be implemented to incapacitate allelic exclusion at the κ or λ light chain locus, wherein the bispecific antibodies produced from an individual B cell consists of heavy chain homodimers or heterodimers paired with two different light chains.

In another specific aspect, analogous strategies to those for heavy chain allelic inclusion are employed to produce bispecific antibodies consisting of heavy chains only. In this specific aspect, the exon encoding the first heavy chain constant domain (C_{H}1) is removed from each allele. Further genetic modifications may be introduced to both heavy chain alleles such that the proteins they express favor heterodimerization with each other and are less compatible with self-dimerization. Alternatively, the modifications may be introduced to the heavy chain alleles such that the C_{H}1-less heavy chain alleles are expressed sequentially in an inducible manner as described in the preceding aspect so as to produce bispecific heavy chain-only antibodies.

In yet another alternative aspect, the immunoglobulin alleles are modified for permissive bispecific antibody production by B cells in animals that are deficient of one or more signaling molecules necessary for allelic exclusion.

Certain modifications introduced to the immunoglobulin alleles that are conducive to bispecific antibody formation have minimal effects on allelic exclusion. For example, heavy chains containing the modifications analogous to the well-known "knob-into-hole" mutations readily form heterodimers; however, the introduced modifications do not completely suppress homodimer formation if each modified heavy chain is expressed alone. Since only one immunoglobulin allele rearranges at a time, the heavy chain homodimers expressed from the first rearranged allele may retain competence for allelic exclusion. The present methods implement such immunoglobulin allele modifications, which on their own have minimal impact on allelic exclusion, in the context of animals or cells in which allelic exclusion is impaired by deficiency of one or more signaling components of the pre-BCR or BCR.

Thus, according to a specific aspect, there is described a genetically modified animal with compromised immunoglobulin heavy chain gene allelic exclusion enabling selection of B lymphocytes each capable of co-expressing two or more different antigen receptors per cell and/or a bispecific antigen receptor.

In some aspects, exons within one or more of the constant region-encoding parts of the immunoglobulin heavy chain gene of the genetically modified animal are changed such that allelic exclusion does not occur following V(D)J rearrangement in developing B lymphocytes. In some aspects, changes to the immunoglobulin heavy chain gene in the genetically modified animal allow for inducible inactivation and/or activation of expression of one or more of the exons in one or more of the constant region-encoding parts of the immunoglobulin heavy chain gene; part or all of one or more constant region exons of the genetically modified animal are placed in inverted reading frame orientation relative to rearranged V(D)J gene segments in the same immunoglobulin heavy chain gene; and in some aspects a DNA cassette is inserted into the genetically modified animal to prevent expression of the constant region exons from rearranged V(D)J gene segment on the same chromosome.

Specifically described herein is a genetically modified animal that when injected with two different antigens simultaneously, or with one antigen followed by second, different antigen, generates B lymphocytes each capable of co-expressing, or sequentially expressing, two or more different antigen receptors and/or a bispecific antigen receptor. In some aspects, heterodimerization of the two antigen receptors in the B lymphocytes is enabled by a developmental or differentiation event, or can be induced.

Specifically described herein is a genetically modified animal where two rearranged immunoglobulin heavy chain genes in individual B cells in the animal express gene products that do not homodimerize efficiently with each other; and in some aspects, homodimerization of the two different heavy chain gene products does not occur, or is disfavored relative to heterodimerization.

Yet specifically described herein is a genetically modified animal with compromised immunoglobulin light chain gene allelic exclusion enabling selection of B lymphocytes each capable of co-expressing two or more different antigen receptors per cell and/or a bispecific antigen receptor. In some aspects, the constant region-encoding exons within one or more of the animal's immunoglobulin light chain genes are changed such that allelic exclusion does not occur following VJ rearrangement in developing B lymphocytes; and in other aspects, changes to one or more of the genetically modified animal's immunoglobulin light chain genes allow for inducible inactivation and/or activation of expression of constant region-encoding parts of the immunoglobulin heavy chain gene.

Specifically described herein is an immunoglobulin light chain gene in the genetically modified animal, wherein part or all of one or more constant region exons are placed in inverted reading frame orientation relative to rearranged VJ gene segments in the same immunoglobulin light chain gene; an immunoglobulin light chain gene in the genetically modified animal, wherein a DNA cassette is inserted to prevent expression of a constant region exon from the rearranged VJ gene segment on the same chromosome; and an immunoglobulin light chain gene in the genetically modified animal, wherein a constant region exon is modified to be compatible for association with one but not both heavy chain alleles in the same B cell.

Specifically described herein are primary B cells, immortalized B cells, or hybridomas derived from the genetically modified animal.

Specifically described herein is a genetically modified animal, wherein changes to the immunoglobulin heavy chain gene allow for production of bispecific antibodies consisting of heavy chains only.

Other aspects include a part or whole immunoglobulin protein transcribed from the immunoglobulin heavy chain genes from the engineered portion of the genetically modified animal; and part or whole engineered immunoglobulin proteins derived from the cells of the genetically modified animal.

These and other aspects, objects and features of the invention are described in more detail below.

### BRIEF DESCRIPTION OF THE FIGURES

Figure 1 illustrates two typical alleles at the heavy chain locus featuring variable gene segments prior to VDJ recombination.
Figure 2 illustrates modifications to the heavy chain alleles for permissive bispecific antibody production from the concurrent expression of two heavy chain alleles.
Figure 3 illustrates the constant region locales of the heavy chain locus featuring exons for the expression of different antibody classes.
Figure 4 illustrates modifications to the heavy chain alleles for bispecific antibody production by sequential heavy chain expression.
Figure 5 illustrates alternative modifications to the heavy alleles for bispecific antibody production by sequential heavy chain expression.
Figure 6 illustrates modifications to the heavy chain alleles for inducible bispecific antibody production.

### DEFINITIONS

The terms used herein are intended to have the plain and ordinary meaning as understood by those of ordinary skill in the art. The following definitions are intended to aid the reader in understanding the present invention, but are not intended to vary or otherwise limit the meaning of such terms unless specifically indicated.

The term "transgene" is used herein to describe genetic material which has been or is about to be artificially inserted into the genome of a cell, and particularly a cell of a vertebrate host animal.

"Transgenic animal" is meant a non-human animal, usually a mammal such as a rodent, particularly a mouse or rat, although other mammals are envisioned, having an exogenous nucleic acid sequence present as a chromosomal or extrachromosomal element in a portion of its cells or stably integrated into its germ line DNA (i.e., in the genomic sequence of most or all of its cells). Animals other than rodents are not according to the claimed invention.

A "vector" includes plasmids and viruses and any DNA or RNA molecule, whether self-replicating or not, which can be used to transform, transduce or transfect a cell.

"Cell surface" refers to the plasma membrane of the cell, i.e., that part of the cell most directly exposed to extracellular spaces and available for contact both with cells and proteins in the extracellular (including intercellular) space.

A "bispecific antibody" is one that comprises two physically separable antigen-binding surfaces which differ from each other in their antigen specificity. Normal antibodies have two physically separable antigen-binding surfaces that are structurally identical and thus have the same antigen specificity. A preferred version of a bispecific antibody is one that resembles a normal IgG antibody molecule with two physically separable antigen-binding surfaces, but instead of these surfaces being structurally identical, they differ from each other. In the context described herein, both of these surfaces may be comprised of the same heavy chain protein but they would differ from each other in the light chain proteins they comprise. Alternatively, the two surfaces may be comprised of the same light chain protein but they would differ from each other in the heavy chain proteins they comprise.

As used herein, "productive rearrangement" is a VDJ or VJ rearrangement that is in frame and enables variable region domain translation. The variable domain of a heavy chain or light chain is considered "functional" if it can be expressed in-frame with the downstream constant region exons(s). A heavy chain or light chain protein translated from a productive VDJ or VJ rearrangement, respectively, is referred to as "functional" if it can be expressed on the cell surface.

An immunoglobulin "allele" described herein refers to a chromosome segment at the heavy chain or light chain locus that may include the variable gene segments, an intronic enhancer, constant regions genes, and other sequences of endogenous or exogenous sources.

"Allelic exclusion" refers to the fact that the vast majority of B cells in vertebrate species such as rodents or humans carry a productively rearranged heavy chain gene on only one of two homologous autosomes. Similarly, allelic exclusion at light chain loci would refer to an analogous scenario. In a more general sense, allelic exclusion applies whenever productive V(D)J rearrangement at any heavy or light chain locus inhibits further rearrangement of other heavy or light chain V(D)J gene segments, respectively, no matter where their chromosomal location. For example, if two or more sets of heavy chain VDJ linkage groups are inserted in the same chromosome, productive rearrangement at one of the heavy chain linkage groups prevents further V(D)J rearrangement at any of the other heavy chain linkage groups. The same principle applies to light chain linkage groups. In principle, this type of "allelic" exclusion would occur by the same mechanism as conventional allelic exclusion

"Allelic inclusion" refers to a loss of allelic exclusion, and thus, to the ability of B cells to produce two or more functional heavy chain variable domains and/or two or more functional light chain variable domains.

A genomic "locale" is any region of the genome, preferably a gene, which is associated with one particular functional aspect. The term locale is used here to refer to parts of immunoglobulin loci. For example, it can refer to that part of an immunoglobulin locus that primarily contains one kind of gene segment, such as a V gene segment locale, or a D gene segment locale, or a J gene segment locale, or more broadly, the variable locale, which includes all of the V, D and J gene segments. The constant region locale, is that part of an immunoglobulin locus that contains constant region exons.

### DETAILED DESCRIPTION OF THE INVENTION

The practice of the techniques described herein may employ, unless otherwise indicated, conventional techniques and descriptions of organic chemistry, polymer technology, molecular biology (including recombinant techniques), cell biology, biochemistry, and sequencing technology, which are within the skill of those who practice in the art. Such conventional techniques include polymer array synthesis, hybridization and ligation of polynucleotides, and detection of hybridization using a label. Specific illustrations of suitable techniques can be had by reference to the examples herein. However, other equivalent conventional procedures can, of course, also be used. Such conventional techniques and descriptions can be found in standard laboratory manuals such as Green, et al., Eds. (1999) Genome Analysis: A Laboratory Manual Series (Vols. I-IV); Weiner, Gabriel, Stephens, Eds. (2007), Genetic Variation: A Laboratory Manual; Dieffenbach, Dveksler, Eds. (2003), PCR Primer: A Laboratory Manual; Bowtell and Sambrook (2003), Condensed Protocols from Molecular Cloning: A Laboratory Manual; and Sambrook and Russell (2002), Molecular Cloning: A Laboratory Manual (all from Cold Spring Harbor Laboratory Press); Stryer, L. (1995) Biochemistry (4th Ed.) W.H. Freeman, New York N.Y.; Lehninger, Principles of Biochemistry 3rd Ed., W. H. Freeman Pub., New York, N.Y.; and Berg et al. (2002) Biochemistry, 5th Ed., W.H. Freeman Pub., New York, N.Y.; Nagy, et al., Eds. (2003) Manipulating the Mouse Embryo: A Laboratory Manual (3rd Ed.) Cold Spring Harbor Laboratory Press, Cold Spring Harbor, N.Y., all of which are herein incorporated in their entirety by reference for all purposes.

Note that as used herein and in the appended claims, the singular forms "a," "an," and "the" include plural referents unless the context clearly dictates otherwise. Thus, for example, reference to "an immunoglobulin" refers to one or more such immunoglobulins, and reference to "the method" includes reference to equivalent steps and methods known to those skilled in the art, and so forth.

Unless defined otherwise, all technical and scientific terms used herein have the same meaning as commonly understood by one of ordinary skill in the art to which this invention belongs. All publications mentioned herein are referred to for the purpose of describing and disclosing devices, formulations and methodologies that may be used in connection with the presently described invention.

Where a range of values is provided, it is understood that each intervening value, between the upper and lower limit of that range and any other stated or intervening value in that stated range is encompassed within the present disclosure. The upper and lower limits of these smaller ranges may independently be included in the smaller ranges, subject to any specifically excluded limit in the stated range. Where the stated range includes one or both limits, ranges excluding either or both of those included limits are also included in the present disclosure.

In the following description, numerous specific details are set forth to provide a more thorough understanding of the present invention. However, it will be apparent to one of skill in the art that the present invention may be practiced without one or more of these specific details. In other instances, well-known features and procedures well known to those skilled in the art have not been described in order to avoid obscuring the invention.

### The Disclosure in General

Herein described are methods and compositions for the rapid isolation of bispecific antibodies from animals. B lymphocytes present in the immune system are normally prevented from expressing more than one functional heavy chain and one functional light chain by a process called allelic exclusion. Specifically described herein are modifications to the genomic contents of animals such that allelic exclusion is incapacitated at one or more of the immunoglobulin loci in developing B cells. The methods described herein provide B cells the ability to express more than one functional V_{H} domain and/or more than one functional V_{L} domain per cell.

**In** certain aspects described herein are modifications to the immunoglobulin alleles to impinge on the normal allelic exclusion processes such that an in-frame V(D)J rearrangement of one allele does not inhibit V(D)J rearrangement of the other allele. Certain aspects also implement two general strategies to isolate bispecific antibodies from transgenic animals. The first strategy involves concurrent immunization regimens with two or more antigens of interest. The second strategy involves sequential immunizations with the different antigens of interest.

Allelic exclusion following both heavy and light chain rearrangements requires the trafficking of heavy chains to the cell surface. In a preferred aspect described herein the likelihood that, if improperly folded, newly synthesized heavy chain polypeptides are retained in the ER and lack the ability to mediate allelic exclusion. Following VDJ recombination of the heavy chain-in addition to the requisite V_{H} domain pairing with the surrogate light chain complex-dimerization of the heavy chain polypeptides is required to achieve proper protein folding. Heavy chain homodimerization for all isotypes, except IgM and IgE, is initiated by symmetric protein interactions between two C_{H}3 domains. IgM and IgE heavy chains initiate homodimerization via protein interactions between their C_{H}4 domains.

In one aspect described herein, the heavy chain alleles are modified to limit the extent of isotype switching, with exons encoding the C_{H}3 or C_{H}4 dimerization domains on both alleles being modified to be less compatible with homodimerization but complementary to each other for heterodimerization. The heavy chain polypeptides expressed from in-frame VDJ rearrangements on either allele alone inefficiently achieve proper protein folding, and therefore cannot traffic to the cell surface to mediate allelic exclusion.

In addition to the V_{H}-V_{L} pairing, light chain allelic exclusion requires proper protein folding of the heavy chain C_{H}1 domains, which are prone to misfolding (see, e.g., Feige, et al., Mol Cell 34: 569-579 (2009)). To achieve proper protein folding, each heavy chain C_{H}1 domain must associate with a constant region of the light chain (Cκ or Cλ). In an extended aspect of the methods described herein, genetic modifications to the exons encoding the C_{H}1 domains of both heavy chain alleles as well as the exons of the light chain constant region domains are implemented, such that each light chain can associate with a heavy chain expressed from only one allele and not both.

The transgenic mice harboring the aforementioned C_{H}3 modifications, with or without the aforementioned C_{H}1 modifications, are immunized with the two antigens of interest simultaneously. Repeated immunizations may be employed to maximize clonal expansion of B cells recognizing both antigens. After the immunization regimen has been completed, standard hybridoma or other techniques are employed to isolate B cells producing bispecific antibodies to both antigens of interest.

According to a specific aspect, a transgenic animal such as a transgenic mouse carries engineered versions of the heavy chain alleles on both chromosomes, wherein modifications are introduced in the heavy chain locales that express the constant domains of the heavy chain molecules. Both engineered alleles are capable of undergoing VDJ rearrangement to create heavy chain diversity during B cell development. One of the engineered alleles is also capable of expressing a full-length transmembrane heavy chain protein that can generate a pre-BCR signal. The other engineered allele is disabled in this regard.

In one aspect described herein, the two engineered alleles carry recognition sequences (wild-type or mutated) for one or more site-specific recombinases such as Cre or Flp. The recognition sites are placed in such a way that site-specific recombination changes the functionality of the constant domain-encoding part of the locus. That is, if the allele is capable of expressing a fully functional heavy chain protein, then site-specific recombination deprives the locus of this property. Similarly, if the allele is incapable of expressing a fully functional heavy chain protein, then site-specific recombination confers the ability to express a functional heavy chain on the locus.

The site-specific recombinase-mediated changes just summarized are accomplished either by deleting or inverting pieces of DNA in the constant domain-encoding part of the heavy chain locus on the two homologous chromosomes. In certain aspects, site-specific recombinase-dependent loss of constant domain full functionality on one chromosome is accompanied by synchronous, or near synchronous, gain of full functionality on the other chromosome. In a favored aspect, expression of this site-specific recombinase is under the control of a promoter from a gene that is induced during B cell activation such as Cγ1 or Aicda (activation-induced deaminase).

The transgenic mice just described are immunized with an antigen allowing for clonal expansion of B cells expressing antibody molecules specific for the antigen. Because of the heavy chain gene modifications they carry, the antibodies specific for this antigen are comprised of heavy chains encoded by rearranged versions of only one of the two heavy chain alleles; namely, the allele defined by full functionality in its constant domain-encoding part. The other allele lacks such functionality and because of this will not encode heavy chains capable of participating in the signaling process necessary for antigen-specific clonal expansion. Repeated immunizations may be employed to maximize clonal expansion and antigen-specific antibody diversity.

After the immunization regimen has been completed, site-specific recombination is induced resulting in a switch of heavy chain constant domain functionality from one chromosome to the other. As a result of this switch, the alleles encoding the heavy chains in antibodies specific for the antigen used in the immunization are deprived of constant domain full functionality. At the same time, or near to it, the alleles that have been previously deprived of constant domain full functionality now gain this functionality. Through this switch in functionality, cells that participate in clonal expansion in response to the antigen used in the immunization regimen gain expression of new heavy chain proteins.

Subsequent to the induced site-specific recombinase-dependent switch just described, the mice are immunized with a second antigen. Clonal expansion in response to the second antigen depends on antibodies comprised of heavy chains encoded by the second allele; i.e., the one that gains constant domain functionality due to the induced site-specific recombinase event. As in the first case, the second immunization may be repeated to maximize clonal expansion and antigen-specific antibody diversity.

The clonally expanded cells expressing antibodies specific for the second antigen include those that have previously been involved in clonal expansion in response to the first antigen. During the second clonal expansion, these cells do not express the heavy chain proteins that have specificity for the first antigen but instead express different heavy chain proteins from the second allele. The heavy chain proteins they express that contribute specificity for the second antigen are encoded by the heavy chain allele that gained constant domain full functionality as a consequence of the induced site-specific recombination event.

After the second immunization regimen has been completed, hybridoma or other techniques are employed to isolate B cells specific for both the first and second antigens. A second site-specific recombinase event may be induced to restore full functionality on the allele that carries antigen specificity for the first antigen. For example, expression of the second site-specific recombinase may be under the control of an inducible system, such as those inducible by Tamoxifen or doxycyline. Through this modification, the capacity of the cells to express two different antibody molecules may be assessed: one specific for the antigen used in the first immunization regimen, and the other specific for the antigen used in the second immunization regimen.

Following the second site-specific recombination, the assembled VDJ exon on each heavy chain allele is now expressed with a protein, or a protein domain, which is a complementary half of a heterodimer (termed a heterodimerizer). In a favored aspect, the heterodimerizers are mutant IgG1 alleles, in which the fourth exons that encode the C_{H}3 domains of both alleles are mutated such that they promote heterodimerization with each other and suppress homodimerization. Alternatively, the heterodimerizer pair may be non-immuloglobulin proteins such as c-Fos and c-Jun (which physiologically heterodimerize to form the AP-1 transcription factor), leucine zippers, or similar proteins that form heterodimers.

In another aspect described herein, rather than allelic exclusion being compromised at the heavy chain locus, it is instead compromised at a light chain locus. In this version, heavy chain allelic exclusion is normal. The light chain allelic inclusion version described herein is conceptually similar to that of the heavy chain allelic inclusion version, featuring analogous modifications in the constant domain-encoding part of the relevant homologous light chain genes. It is exploited using a similar double immunization scheme combined with an appropriately-staged inducible site-specific recombination step. Bispecific B cells are identified and/or isolated in a similar fashion to what has been described for the heavy chain allelic inclusion version described herein. This light chain allelic inclusion version described herein yields bispecific antibodies each comprised of two light chain proteins and one heavy chain protein, whereas the heavy chain allelic inclusion version yields bispecific antibodies each comprised of one light chain protein and two heavy chains.

In an alternative aspect, strategies analogous to those just described for heavy chain allelic inclusion are implemented to generate B cells that produce bispecific antibodies consisting of heavy chains only. Herein described, exons encoding the C_{H}1 domains are removed from both heavy chain alleles. The C_{H}1-less polypeptides expressed from each heavy chain allele should exhibit little, if any, dependence on the presence of light chains or surrogate light chains for proper protein folding as well as trafficking to the cell surface (see, e.g., Feige, et al., Mol Cell 34: 569-579 (2009)). The variable gene segments of both heavy chain alleles may be derived from animals that naturally express single-chain antibodies, such as camelids, or from other animals with or without modifications to improve the thermo-stability of the V_{H} domains when expressed without light chains.

In one specific aspect, the heavy chain alleles are further modified to limit the extent of isotype switching, with exons encoding the C_{H}3 or C_{H}4 dimerization domains on both alleles being mutated to be less compatible with homodimerization but complementary to each other for heterodimerization. The C_{H}1-less heavy chain polypeptides expressed from either mutant allele alone should not traffic to the cell surface to mediate allelic exclusion because the mutations suppress efficient heavy chain homodimerization. Therefore, successful B cell development depends on the concurrent expression of C_{H}1-less heavy chains from both mutant heavy chain alleles.

Transgenic mice lacking the aforementioned C_{H}1 domain-encoding exons and harboring the aforementioned C_{H}3 modifications are immunized with the two antigens of interest simultaneously. Repeated immunizations may be employed to maximize clonal expansion of B cells recognizing both antigens. After the immunization regimen has been completed, standard hybridoma or other techniques are employed to isolate B cells producing bispecific antibodies to both antigens of interest.

In an alternative version of this specific aspect, allelic inclusion of heavy chains lacking C_{H}1 domains is achieved via sequential expression of each mutant heavy chain allele in an inducible manner analogous to the heavy chain allelic inclusion schemes described in the preceding aspect. The heavy chain alleles are also modified such that an in-frame VDJ rearrangement on one allele is incapacitated for allelic exclusion and preserved for expression at a later B cell developmental stage. This is similarly achieved via disrupting the open reading frame of one heavy chain allele by a DNA cassette or by inversion of one or more exons that encode the heavy chain constant region. An inducible event of site-specific DNA recombination is likewise introduced to alternate the expression of each mutant heavy chain allele. A similar double immunization scheme followed by a second inducible site-specific recombination step is employed to obtain bispecific B cells. This heavy chain allelic inclusion version described herein yields bispecific antibodies each comprised of two heavy chains without light chains.

In other alternative aspects, the immunoglobulin alleles that are modified for permissive bispecific antibody production by B cells are implemented in animals that are deficient of one or more signaling molecules necessary for allelic exclusion.

In certain cases, the methods introduce modifications to the immunoglobulin genes that are designed to be conducive to bispecific antibody formation, but have minimal effects on allelic exclusion. In one specific aspect, heavy chains containing modifications analogous to the well-known "knob-into-hole" mutations are introduced to the exons encoding the C_{H}3 domain of the heavy chains (see, e.g., Ridgway, et al., Protein Eng 9: 617-621 (1996)). The introduced modifications do not completely suppress homodimer formation if each modified heavy chain is expressed alone. As only one immunoglobulin allele rearranges at a time, the heavy chain homodimers expressed from the first rearranged allele may retain competence for allelic exclusion. The present disclosure implements such immunoglobulin allele modifications, which on their own have minimal impact on allelic exclusion, in animals or cells that have impaired allelic exclusion caused by the deficiency of one or more signaling components of the pre-BCR or BCR. One example of such mutants is the mouse strain deficient of E2A (see, e.g., Hauser, et al., Journal of Immunology 192:2460-2470 (2014)).

The transgenic mice harboring the aforementioned C_{H}3 modifications and a defective pre-BCR or BCR signaling component are immunized with the two antigens of interest simultaneously. Repeated immunizations may be employed to maximize clonal expansion of B cells recognizing both antigens. After the immunization regimen has been completed, standard hybridoma or other techniques are employed to isolate B cells producing bispecific antibodies to both antigens of interest.

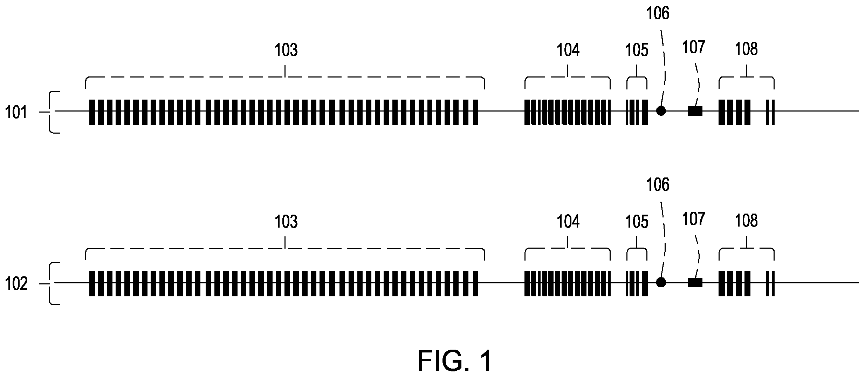

Figure 1 depicts two heavy chain alleles (101 and 102) typically found in humans and most vertebrate animals. Each heavy chain allele consists of multiple V (103), D (104), and J (105) genes upstream of the Cµ exons that encode the constant regions of IgM (108). On each chromosome, regulatory elements such as the 5' "intronic" enhancer (106) and switch region (107) also exist between the J genes and the first C_{H} exon. In the preferred aspects, the V, D, and J gene segments of the transgenic mice are chimeric, consisting of human coding regions and mouse noncoding regions. Labeled components in the figure are as follows: Chromosomal segments of the heavy chain locus (101, 102); Variable region gene segments (103); D region gene segments (104); J region gene segments (105); Heavy chain "intronic" enhancer (106); Switch region (107); Cµ exons encoding the constant regions of IgM (108).

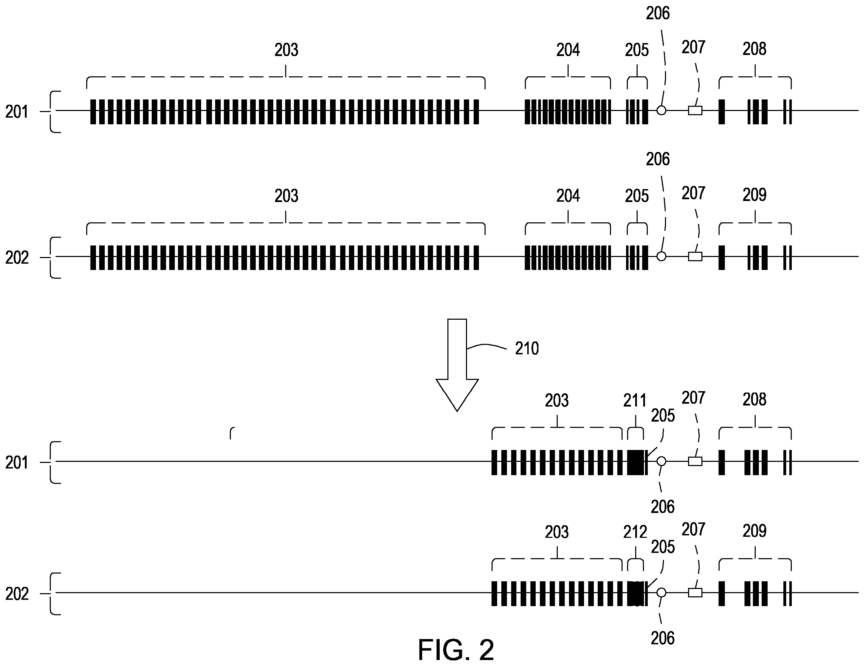

Figure 2 depicts an example of modifications that the present disclosure implements to both heavy chain alleles (201, 202) in order to render B cells permissive for allelic inclusion. Under germline configuration, each heavy chain allele consists of unrearranged V (203), D (204), and J (205) genes followed by modified Cγ exons encoding an IgG isotype (208 and 209). In the preferred aspects, the V, D, and J gene segments of the transgenic mice are chimeric, consisting of human coding regions and mouse noncoding regions. During B cell development, randomly selected V, D, and J genes assemble (210) to form the V_{H} exons (211, 212). Certain codons within the C_{H}3 exons of each heavy chain allele are mutated such that the encoded heavy chains are compatible with heterodimerization with each other (such as mutant Cγ1, whose cDNA sequences for the two heavy chain alleles are specified at [SEQ ID No. 1 and 2]). Additionally, the modifications cause the translated heavy chain polypeptides to misfold if either allele is expressed alone. Consequently, pre-BCRs cannot form and traffic to the plasma membrane to mediate allelic exclusion, unless the mutant heavy chain polypeptides form heterodimers from the concurrent expression of both alleles. In an extended version of this specific aspect, in combination with the aforementioned C_{H}3 mutations, certain codons within the C_{H}1 exons of both heavy chain alleles are similarly modified such that each heavy chain can be paired with a different light chain. Although this figure exemplifies the use of mutant IgG heavy chains to suppress allelic exclusion as well as to facilitate heavy chain heterodimerization, other isotypes (i.e., IgM, IgD, or IgA) could be similarly employed. Labeled components in the figure are as follows: Chromosomal segments of modified heavy chain alleles (201, 202); Variable region gene segments (203); D region gene segments (204); J region gene segments (205); Heavy chain "intronic" enhancer (206); Switch region (207); Cγ exons encoding mutant constant regions of IgG (208, 209); VDJ recombination events (210); Assembled VDJ exons (211, 212).

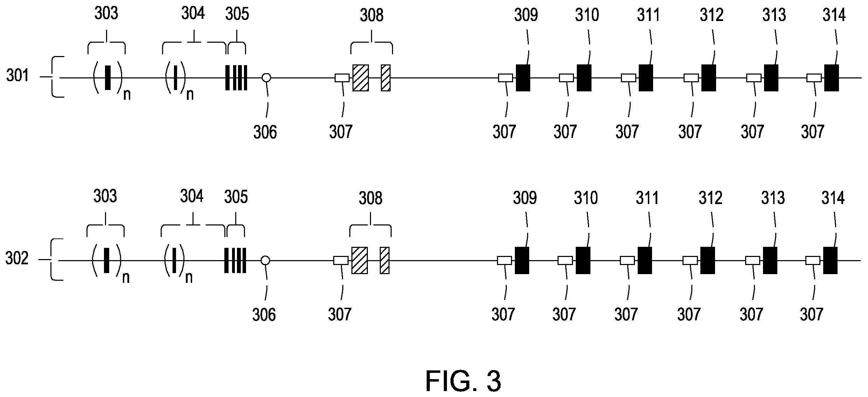

Figure 3 depicts the heavy chain genes on two homologous chromosomes found in rodents. (The typical human heavy chain locus is quite similar but has more constant region genes.) In this figure, the V (303) and D (304) genes of both heavy chain alleles (301, 302) are compressed (denoted by "n") compared to Figure 1 so as to emphasize the structure of the constant region locales of the chromosomes. In the preferred aspects, the V (303), D (304), and J (305) gene segments of the transgenic mice are chimeric, consisting of human coding regions and mouse noncoding regions. IgM and IgD (308, individual C_{H} exons not shown) are the first isotypes to be expressed by B cells. The C_{H} exons encoding other antibody classes (309-314, individual exons not shown) exist further downstream. In the C57BL/6 mouse strain, these are Cγ3 (309), Cγ1 (310), Cγ2b (311), Cγ2c (312), Cε (313), and Cα (314). Certain mouse strains such as BALB/c have Cγ2a instead of Cγ2c. Except for that of Cδ, an isotype switch region (307) is present preceding the first C_{H} exon of each antibody class. Labeled components in the figure are as follows: Heavy chain alleles (301, 302); V genes denoted in brackets, "n" referring to multiple number of the gene segments (303); D genes denoted in brackets, "n" referring to multiple number of the gene segments (304); J gene (305); Heavy chain "intronic" enhancer (306); Switch region (307); Cµ and Cδ (308, individual exons not shown); C_{H} exons of IgGs and other antibody classes (309-314, individual exons not shown).

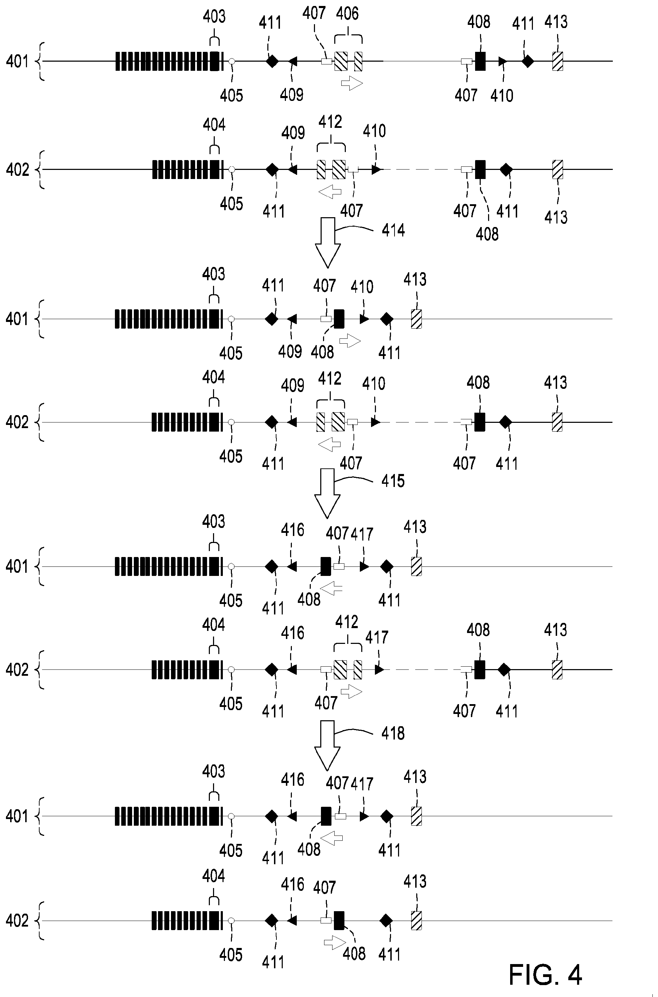

Depicted in Figure 4 are two heavy chain alleles (401, 402) after VDJ rearrangements have occurred to produce different V_{H} exons (403, 404), as they would be found in naive B cells. On allele 401, the exons encoding Cµ and/or Cδ (406) are in the same sense orientation (arrow below) as the rearranged VDJ exon (403). By contrast, the Cµ and/or Cδ exons (412) are present in the reverse orientation (arrow below) relative to the VDJ exon (404) on allele 402. Thus, allele 402 is not capable of expressing full-length heavy chain polypeptides as its Cµ and/or Cδ exons are in the antisense configuration, and normal B cell development is dependent on the expression of functional heavy chains from allele 401. The inverted Cµ and/or Cδ exons (412) in allele 402 are immediately flanked by two oppositely oriented recognition sequences (409, 410) for site-specific DNA recombination. In allele 401, the two recognition sequences for site-specific DNA recombination (409, 410) are placed further apart, also in opposite orientation: one (409) upstream of the Cµ and/or Cδ exons (406), the other (410) downstream of Cγ exons (408). During a B cell response to an immunogen (414), allele 401 undergoes isotype switching, which brings the downstream site-specific DNA recombination sequence (410) closer to its upstream counterpart (409). When expression of the site-specific DNA recombinase is induced (415), the DNA segments flanked by its recognition sequences (409, 410) on both alleles undergo irreversible inversion because the resultant recombination sites (416, 417) are no longer competent for recombination. Allele 402 is now capable of expressing full-length heavy chains, while allele 401 becomes inactivated by the inversion of its C_{H} exons. Following immunization with a second antigen (418), allele 402 undergoes isotype switching. The invertible DNA segments of both alleles are additionally flanked by recognition sequences (411) for a second site-specific DNA recombinase, which can be introduced before or after hybridoma generation. Following excision of the intervening DNA fragments, the VDJ exons on both alleles (403, 404) are now expressed with the downstream C_{H} exons modified to be compatible with heavy chain heterodimerization (413). Labeled components in the figure are as follows: Heavy chain alleles (401, 402); Assembled VDJ exons (403, 404); Heavy chain "intronic" enhancer (405); C *µ* and/or Cδ exon(s) (406) initially in sense orientation (arrow below) relative to assembled VDJ exon (403); Switch region (407); C_{H} exons of switched isotype (408); Recognition sequences for the first site-specific recombinase (409, the sequence of this site is in opposite orientation to the site labeled 410); Recognition sequence for the second site-specific recombinase (411); Cµ and/or Cδ exon(s) (412) initially in reverse orientation (arrow below) relative to assembled VDJ exon (404); Modified exons compatible with heavy chain heterodimerization (413); Antigen response followed by isotype switching (414); First site-specific DNA recombinase expression (415); Remnant DNA sequences following site-specific DNA recombination (416, 417); Antigen response to second immunogen and isotype switching (418).

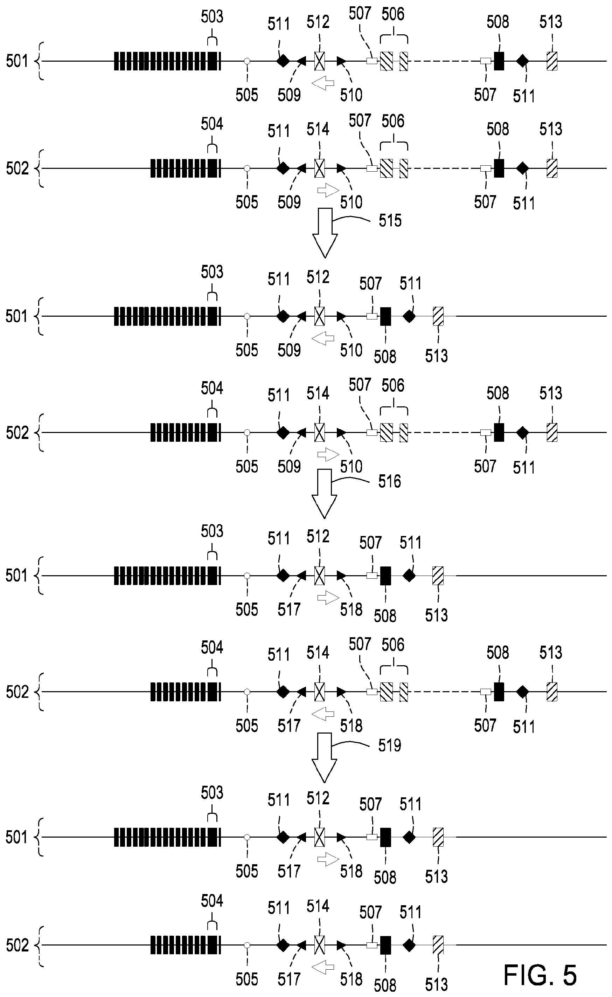

Figure 5 illustrates alternative modifications to the heavy alleles for bispecific antibody production by sequential heavy chain expression. Shown are two heavy chain alleles (501, 502) after VDJ rearrangements have occurred to produce different V_{H} exons (503, 504), as they would be found in naive B cells. On allele 501, a DNA cassette (512) flanked by two oppositely oriented recognition sequences (509, 510) for a site-specific DNA recombinase is inserted downstream of the J genes before the exons encoding Cµ and/or Cδ constant domains (506). The DNA cassette (512) is in reverse orientation (arrow below) relative to the assembled VDJ exon (503) and contains one or more of the following: a splice acceptor, a ribosomal skip sequence or IRES, an open reading frame, a stop codon, or a poly-adenylation signal sequence. A similar DNA cassette (514) is also inserted at an analogous locale on allele 502, but is in the same sense orientation (arrow below) as the rearranged VDJ exon (504). Allele 502 cannot express full-length heavy chains because the DNA cassette (514) disrupts its open reading frame following the VDJ exon (504). By contrast, allele 501 can express full-length heavy chains because the inverted DNA cassette does not disrupt its open reading frame. Downstream of the IgG exons of both alleles is a recognition site (511) for a second DNA recombinase. Also on both alleles, another site for the second DNA recombinase (511) is inserted upstream of the recognition sites for the first DNA recombinase (509). During a B cell response to an immunogen (515), allele 501 undergoes isotype switching. When expression of the first site-specific DNA recombinase is induced (516), the DNA cassettes on both alleles (512, 514) undergo irreversible inversion because the resultant recombination sites (517, 518) are no longer competent for recombination. Allele 502 is now capable of expressing full-length heavy chains because the DNA cassette (514) is now in reverse orientation and no longer disrupts the heavy chain open reading frame. By contrast, the open reading frame of allele 501 is disrupted by the DNA cassette (512) in the same orientation as its VDJ exon (503). Following immunization with a second antigen (519), allele 502 undergoes isotype switching. When expression of the second site-specific DNA recombinase is introduced before or after hybridoma generation, the intervening DNA fragments (512, 514) are now excised at its cognate recognition sequences (511). Subsequently, the VDJ exons on both alleles (503, 504) are now expressed with C_{H} exons modified for heavy chain heterodimerization (513). Labeled components in the figure are as follows: Heavy chain alleles (501, 502); Assembled VDJ exons (503, 504); Heavy chain "intronic" enhancer (505); Cµ and/or Cδ exons (506); Switch region (507); C_{H} exons of switched isotype (508); Recognition sequences for the first site-specific recombinase (509, the sequence of this site is in opposite orientation to the site labeled 510); Recognition sequence for the second site-specific recombinase (511); DNA cassette (512) initially in reverse orientation (arrow below) relative to VDJ exons (503); Modified exons compatible with heavy chain heterodimerization (513); DNA cassette (514) initially in sense orientation (arrow below) relative to VDJ exon (504); Antigen response followed by isotype switching (515); First site-specific DNA recombinase expression (516); Remnant DNA sequences following site-specific DNA recombination (517, 518); Antigen response to second immunogen and isotype switching (519).

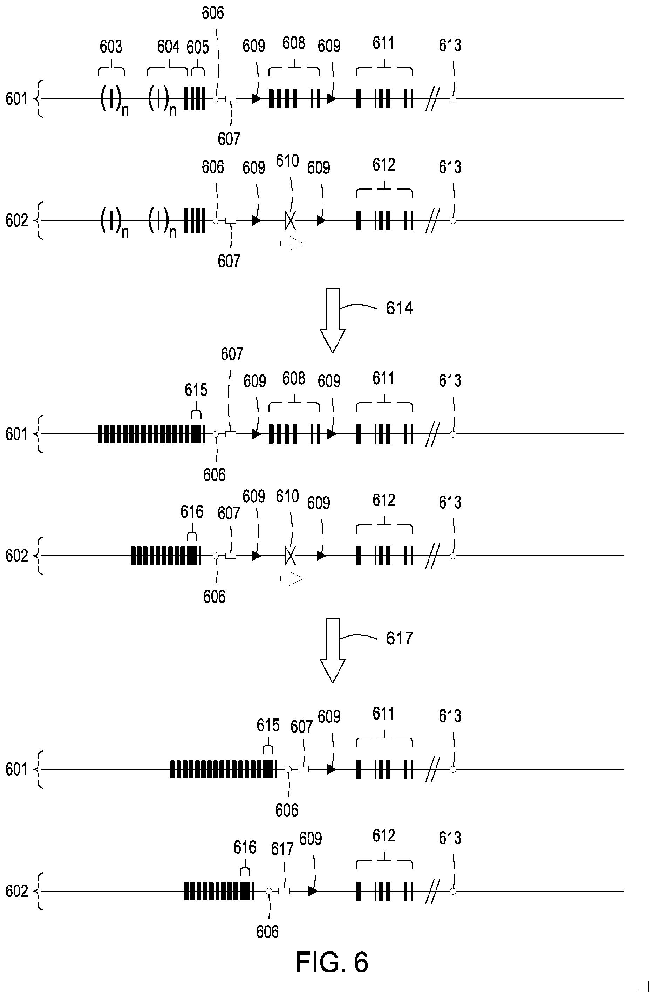

Figure 6 illustrates modifications to the heavy chain alleles for inducible bispecific antibody production. In germline configuration, one heavy chain allele (601) consists of unrearranged V (603), D (604) and J (605) genes followed by the Cµ and/or Cδ exons (608) that are flanked by two recognition sequences for a site-specific DNA recombinase (609). On the other heavy chain allele (602), a DNA cassette (610) in sense orientation (arrow below) flanked by two recognition sequences for the same site-specific DNA recombinase (609) is inserted downstream of the V, D, and J genes. In the preferred aspects, the V, D, and J gene segments of the transgenic mice are chimeric, consisting of human coding regions and mouse noncoding regions. The DNA cassette contains one or more of the following: a splice acceptor, a ribosomal skip sequence or IRES, an open reading frame, a stop codon, or a poly-adenylation signal sequence. Additionally, the heavy chain alleles contain the same modifications to the C_{H} exons for heterodimerization (611 and 612) as those described in Figure 2 (208 and 209 in Figure 2). Following VDJ recombination events (614), successful assembly of an in-frame V_{H} exon (615) on allele 601 results in normal expression of full-length heavy chains. By contrast, the DNA cassette (610) on allele 602 prevents any successful VDJ rearrangement (616) from being able to express full-length heavy chain polypeptides. Upon expression of a site-specific DNA recombinase gene (617), C_{H} exons (608) on the first allele (601) as well as the DNA cassette (610) on the second allele (602) are excised from the chromosomes. VDJ exons on both alleles (615, 616) can now be expressed with downstream C_{H} exons containing modifications conducive to heavy chain heterodimerization. Labeled components in the figure are as follows: Modified heavy chain alleles (601, 602); V genes denoted in brackets, with "n" referring to the presence of multiple gene segments (603); D genes denoted in brackets, with "n" referring to the presence of multiple gene segments (604); J genes (605); Heavy chain "intronic" enhancer (606); Switch region (607); Cµ and/or Cδ exons (608); Recognition sequences for site-specific DNA recombinases (609); DNA cassette in sense orientation indicated by arrow below (610); Mutant Cγ1 exons (611; 612); Heavy chain 3' enhancer (613); VDJ recombination events (614); Assembled VDJ exons (615, 616); Activation of site-specific DNA recombinase (617).

### Transgenic Cell Libraries

The transgenic cells described herein may be used to produce expression libraries, preferably low complexity libraries, for identification of antibodies of interest on the surface of plasma cells. The present disclosure thus also includes antibody libraries produced using the cell technologies described herein for identification of antigen-specific antibodies expressed on plasma cells.

### Transgenic Animals

In specific aspects, herein provided are transgenic animals carrying engineered in heavy chain or light genes.

In some specific aspects, the transgenic animals described herein further comprise human immunoglobulin regions. For example, numerous methods have been developed for replacing endogenous mouse immunoglobulin regions with human immunoglobulin sequences to create partially- or fully-human antibodies for drug discovery purposes. Examples of such mice include those described in, for example, U.S. Pat Nos. 7,145,056; 7,064,244; 7,041,871; 6,673,986; 6,596,541; 6,570,061; 6,162,963; 6,130,364; 6,091,001; 6,023,010; 5,593,598; 5,877,397; 5,874,299; 5,814,318; 5,789,650; 5,661,016; 5,612,205; and 5,591,669.

In a particularly preferred aspect, the transgenic animals described herein comprise chimeric immunoglobulin segments as described in co-pending application US Pub. No. 2013/0219535 by Wabl and Killeen. Such transgenic animals have a genome comprising an introduced partially human immunoglobulin region, where the introduced region comprising human variable region coding sequences and non-coding variable sequences based on the endogenous genome of the non-human vertebrate. Preferably, the transgenic cells and animals described herein have genomes in which part or all of the endogenous immunoglobulin region is removed.

### Use in Antibody Production

Culturing cells *in vitro* has been the basis of the production of numerous therapeutic biotechnology products, and involves the production of protein products in cells and release into the support medium. The quantity and quality of protein production over time from the cells growing in culture depends on a number of factors, such as, for example, cell density, cell cycle phase, cellular biosynthesis rates of the proteins, condition of the medium used to support cell viability and growth, and the longevity of the cells in culture. (See, for example, Fresney, Culture of Animal Cells, Wiley, Blackwell (2010); and Cell Culture Technology for Pharmaceutical and Cell-Based Therapies, Ozturk and Ha, Eds., CRC Press, (2006).)

Herein provided is a source of B cells derived from immunization schemes in which animals are challenged with two or more antigens simultaneously, or first with one antigen and then later with another antigen. In both cases, multiple immunizations may be employed to increase antigen-specific antibody titers in individual animals. An inducible site-specific recombination step is included between the two immunization series. Subsequent to the final immunization scheme, B cells are isolated and cultured or used to create hybridomas, or used as a source of RNA for cloning immunoglobulin chain genes. The B cells or the antibody chains they contain are tested for bispecific antigen-binding properties. In the case of hybridomas, this is accomplished by screening hybridomas directly for bispecific antibodies, or for specificity for one kind of antigen and then further analyzing them for whether they carry additional rearranged immunoglobulin chain genes that confer specificity for a second kind of antigen, i.e., they have latent or expressed bi-specificity.

### EXAMPLES

The following examples are put forth so as to provide those of ordinary skill in the art with a complete disclosure and description of how to make and use the present invention, and are not intended to limit the scope of what the inventors regard as their invention, nor are they intended to represent or imply that the experiments below are all of or the only experiments performed.

Efforts have been made to ensure accuracy with respect to terms and numbers used (e.g., vectors, amounts, temperature, etc.) but some experimental errors and deviations should be accounted for. Unless indicated otherwise, parts are parts by weight, molecular weight is weight average molecular weight, temperature is in degrees centigrade, and pressure is at or near atmospheric.

**Example 1. Engineered heavy chain alleles permissive for the isolation of bispecific antibodies following simultaneous immunization with two or more antigens.** Transgenic mice are generated carrying two modified heavy chain alleles that lack the ability to isotype switch (as depicted in Figure 2). Both alleles can undergo VDJ recombination. In a preferred embodiment, an IgG1 instead of IgM heavy chain is expressed during B cell development, but any other isotype including IgM may be employed. B cells expressing only IgG1 develop quite normally and respond to antigens during immunization (see, e.g., Waisman, et al., Journal of Experimental Medicine 204:747-758 (2007)).

The fourth exons of both IgG1 alleles, which encode the C_{H}3 domains, are mutated such that they promote the formation of heavy chain heterodimers and suppress homodimerization. In preferred methods, the mutations are D276K, E233K, and Q234K on one heavy chain allele; and K286D, K269D, and T247D on the other heavy chain allele (amino acid numbering starts at the first codon of C_{H}1). The mutations at similar positions in the human IgG1 heavy chain have been shown to promote heterodimerization and the secretion of bispecific antibodies in cell lines (see, e.g., Gunasekaran, et al., Journal of Biological Chemistry 285:19637-19646 (2010)).

During B cell development, pro-B cells that have rearranged only one heavy chain allele are blocked in development, because the mutant IgG1 heavy chains inefficiently homodimerize to form pre-BCRs. Therefore, insufficient signals are transduced to mediate allelic exclusion, and the second heavy chain allele expressing mutant Cγ1 becomes permissive for VDJ recombination. Developing B cells can progress beyond the pro-B cell stage only when both mutant heavy chains are co-expressed and heterodimerize to form pre-BCRs.

Ultimately, mature B cells in the transgenic mice of the preferred methods harbor two functional heavy chains per cell with one light chain. The transgenic mice may be used for simultaneous immunization with two or more antigens of interest with the subsequent generation of hybridomas using standard methodologies. Hybridomas derived from the transgenic mice are permissive for the direct isolation of bispecific antibodies.

In specific aspects of the heavy chain alleles for this Example 1, the V (203), D (204), and J (205) genes of both heavy chain alleles (201, 202) comprise human coding sequences with mouse regulatory sequences and are described in the co-pending application US Pub. No. 2013/0219535 by Wabl and Killeen. Sequences of the heavy chain "intronic" enhancer (206) as well as all downstream elements, including the heavy chain constant region genes, found in a wild-type mouse are described in LOCUS: NG_005838 (1..180,971). In this Example 1, the sequence spanning 8,608..175,134 containing the C_{H} exons of all isotypes is deleted from both heavy chain alleles and is replaced by modified Cγ1 exons (elements 208 and 209 in Figure 2). Exon 4 of both elements 208 and 209 are mutated to favor heterodimerization with each other over dimerization with self. The mutant Cγ1 cDNA sequences of both heavy chain alleles are specified at [SEQ ID No. 1 and 2], as well as the wild type cDNA sequence of the heavy chain allele [SEQ ID No.3].

### Example 2. Engineered heavy chain alleles permissive for the isolation of bispecific antibodies following sequential immunizations.

Transgenic mice are generated carrying heavy chain alleles that can be switched on or off by site-specific DNA recombination. One of the alleles is capable of expressing full-length heavy chains after a productive VDJ rearrangement, while the other allele lacks this functionality.

Both alleles contain recognition sequences for one or more site-specific recombinases, positioned within the constant domain-encoding locale. Site-specific recombination at these sites causes an inversion of a piece of DNA in both alleles, and as a consequence of this inversion, the constant domain functionality in the alleles is changed.

Site-specific recombination confers the capacity to express a full-length heavy chain protein on the allele that initially lacked this capacity. By contrast, site-specific recombination removes this capacity from the allele that initially has it.

A specific aspect is depicted in Figure 4, with the relevant site-specific recombination sequences marked as 409 and 410, and with the constant domain-encoding exons labeled as 406 and 412. The transcriptional orientation of the constant domain-encoding exons is depicted by the arrow immediately below the labeled components.

Figure 4 shows the two alleles in their configuration after VDJ rearrangements have occurred to generate the V_{H} exons (403 and 404). However, VDJ rearrangements of the alleles depicted in Figure 4 could result in nonproductive gene segment joins on either allele. Similarly, the process could also result in productive rearrangements on either allele. B cells only complete their development in the bone marrow if they express full-length heavy chain molecules from allele 401, because even though allele 402 can undergo a productive VDJ recombination, it cannot express full-length heavy chains since all or part of the Cµ and/or Cδ exons are in the reverse orientation.

Peripheral B cells that develop in the mice carrying the alleles shown in Figure 4 express B cell antigen receptors (transmembrane or secreted) comprised of heavy chains derived from allele 401. The light chains in these B cell antigen receptors derive from normal independent VJ rearrangements at one of their light chain loci.

Immunization of mice carrying the alleles shown in Figure 4 result in clonal expansion of B cells expressing antibodies specific for the immunogen. Crucially, once again, these antibodies are solely comprised of heavy chains derived from allele 401. Repeated immunizations result in enhanced clonal expansion, somatic hypermutation, and isotype switching similar to what occurs in normal mice (shown as 414 in Figure 4).

Transgenic mouse systems exist, or can be readily engineered, to permit inducible expression of particular site-specific DNA recombinases in multiple cell types including B lymphocytes. In yet another aspect of the present methods, transgenic mice carrying the mutant alleles 401 and 402 depicted in Figure 4 additionally harbor an inducible site-specific recombinase system, such as the tamoxifen-inducible system. Immunized mice that have made demonstrable antibody responses to the antigen used in the immunization are caused to express the relevant site-specific recombinase.

Following the expression of a site-specific DNA recombinase (415 in Figure 4), the chromosomal segments flanked by sites for the induced DNA recombinases undergo inversion on both alleles. A resultant feature is the loss of capacity to express full-length heavy chains on allele 401. Concurrently, allele 402 gains the capacity to express full-length heavy chains.

Allele 402 is productively rearranged in some of the B cells that have undergone clonal expansion in response to the immunization. The frequency of such second allele productive rearrangements is typically much higher than is normally the case because this allele does not express full-length heavy chains during B cell development, yet it nonetheless is capable of undergoing VDJ rearrangements.

Immunization of mice carrying the newly activated allele 402 depicted in Figure 4 results in clonal expansion of B cells expressing B cell antigen receptors specific for the antigen used in the immunization. These B cell antigen receptors (transmembrane or secreted) are comprised solely of heavy chains derived from allele 402. Repeated immunizations result in enhanced clonal expansion, somatic hypermutation, and isotype switching similar to what occurs in normal mice.

B cells specific for the antigen used in the second immunization include some that have not undergone clonal expansion in response to the first antigen. Such B cells are not a desirable source for bispecific antibodies capable of recognizing the antigens used in both of the immunizations.

However, some fraction of the B cells specific for the second antigen have been involved in clonal expansion in response to the first antigen. These B cells are an obvious source for bispecific antibodies since one of their rearranged heavy chain genes carries specificity for the first immunogen, while their other rearranged heavy chain genes carries specificity for the second immunogen. In both cases, individual B cells pair one light chain protein with both heavy chain proteins.

Hybridoma or other cloning technology may be exploited to recover B cells with specificity for the second immunizing antigen. These B cells are then analyzed to determine whether they also carry a rearranged heavy chain gene that confers specificity for the first immunizing antigen.

**Example 3. Alternative engineered heavy chain alleles permissive for the isolation of bispecific antibodies following sequential immunizations.** The methods described here are very similar to those described in Example 2. Transgenic mice are engineered to carry two heavy chain immunoglobulin alleles that can be switched on or off by a site-specific DNA recombinase system. The two alleles are designed for sequential immunization schemes similar to those described in Example 2, and consequently feature largely the same kind of functionality in their constant domain locales. Where the alleles differ is in the inclusion of elements designed to improve the efficiency with which the desired kind of bispecific B cells are isolated.

This aspect is depicted in Figure 5. On one of the two engineered heavy chain alleles (allele 501), a DNA cassette (512) flanked by two recognition sequences (509 and 510) for a site-specific DNA recombinase is inserted after the heavy chain enhancer (505) but before the switch region (507) preceding the Cµ exons (506), so that the heavy chain enhancer (505) remains in the genome after isotype switching. On the second heavy chain allele (allele 502), similar elements are inserted at an analogous position, but in the opposite orientation (arrow below). The DNA cassettes are designed to disrupt the open reading frame of the heavy chain exons when aligned in the same transcriptional orientation as the assembled VDJ exon.

Specifically, as described herein, allele 501 consists of exons 15 and 16 from the murine integrin beta-7 (*Itgb7*) gene, aligned in the opposite orientation from the V_{H} exon. Both *Itgb7* exons contain a splice acceptor. Additionally, *Itgb7* exon 15 harbors a stop codon, while *Itgb7* exon 16 contains a stop codon as well as a poly-adenylation sequence signal. Because of the inverted transcriptional orientation, this DNA cassette does not interfere with heavy chain expression from an in-frame VDJ recombination on allele 501.

In a preferred configuration, the inserted DNA cassette in allele 502 consists of an open reading frame from a gene that provides survival, functional, or selection advantages to the B cells that have successfully assembled an in-frame V_{H} exon from VDJ recombination. An example of such gene is the anti-apoptotic B-cell lymphoma-2 (*Bcl2*). The open reading frame of the advantageous gene is aligned in the same transcriptional orientation as the heavy chain mRNA. To prevent this gene from being expressed as a protein fused to the V_{H} exon, a ribosomal skip sequence such as the 2A peptide from a picornavirus is placed between the splice acceptor and the open reading frame of the advantageous gene. The 2A peptide also ensures that the advantageous gene is only expressed in the B lymphocytes that have successfully assembled an in-frame V_{H} exon, and not in the B cells that lack a productive VDJ rearrangement.

According to a specific aspect, the assembled VDJ genes (503, 504) on both heavy chain alleles (501, 502) are derived from individual gene segments comprising human coding sequences with mouse regulatory sequences and are described in the co-pending application US Pub. No. 2013/0219535 by Wabl and Killeen. All endogenous sequences downstream, including the heavy chain constant region genes, are described in LOCUS: NG_005838 (1..180,971). Sequences of the *Itgb7* and *Bcl2* DNA cassettes (elements 512 and 514, respectively) are specified at [SEQ ID Nos. 4 and 5] and are inserted at around position 178,000 of the locus, in between the heavy chain "intronic" enhancer and the switch region of the first exon Cµ exon. In this aspect, recognition sequences for the first site-specific DNA recombinase (elements 509 and 510) are lox66 and lox71 (see, e.g., Oberdoerffer, et al., Nucleic Acids Res 31:e140 (2003)). Also in this aspect, recognition sequences for the second site-specific DNA recombinase (elements 511) are those used by Flp enzyme (see, e.g., McLeod, et al., Mol Cell Biol 6:3357-3367 (1986)). Specifically described herein, the heterodimers (element 513 on each heavy chain allele) are mutant Cγ1 designed to favor heterodimerization with each other over self-dimerization. The mutant Cγ1 cDNA sequences of both heavy chain alleles are specified at [SEQ ID Nos. 1 and 2].

As in Example 2, following the first immunization, allele 501 can respond normally to the antigen because the inverted *Itgb7* cassette has no effect on the transcriptional activities of the heavy chain locus. Because the second DNA cassette interrupts the heavy chain open reading frame of allele 502, no specificity for the immunogen is selected from allele 502 following the first immunization.

Upon expression of a site-specific DNA recombinase (516), the DNA cassettes on both heavy chain alleles are inverted. The open reading frame of the *Itgb7* gene cassette in allele 501 is now in the same transcriptional orientation as the heavy chain mRNA. Effectively, the stop codons and poly-adenylation signal sequence of the *Itgb7* gene prevents the expression of full-length heavy chains from allele 501.

By contrast, allele 502 can now express full-length heavy chains because the open reading frame of the inserted advantageous gene is no longer in the same orientation as the heavy chain mRNA. Allele 502 subsequently can respond to the immunogen in the second the immunization.

Hybridoma or other cloning technology may be exploited to recover B cells with specificity for the second immunizing antigen. These B cells can then be analyzed to determine whether they also carry a rearranged heavy chain gene that confers specificity for the first immunizing antigen.

**Example 4. Alternative engineered heavy chain alleles permissive for the isolation of bispecific antibodies following simultaneous immunization with two or more antigens.** The scheme for this Example 4 is depicted in Figure 6 and contains some elements of both Example 1 as well as Example 3. Transgenic mice are engineered to carry two modified heavy chain alleles that lack the ability to isotype switch by normal means. On one heavy chain allele (601 in Figure 6), the Cµ and/or Cδ heavy chain exons are flanked by directly-oriented recognition sequences for a site-specific recombinase. Further downstream are Cγ1 exons containing C_{H}3 mutations for inter-heavy chain heterodimerization as described for allele 201 in Figure 2 and Example 3, such as the mutant Cγ1 cDNA sequences for two heavy chain alleles as specified at [SEQ ID No. 1 and 2]. Allele 601 can support normal B cell development because its open reading frame transcribed from the assembled V_{H} exon is uninterrupted by the recognition sequences for the site-specific DNA recombinase. On the second heavy chain allele (602 in Figure 6), a DNA cassette (610) flanked by the directly-oriented recognition sequences (609) for the same site-specific DNA recombinase is inserted downstream of the J genes (605). Thus, an in-frame VDJ assembly on allele 602 is deprived of the capacity to express full-length heavy chains because the DNA cassette (610) is designed to disrupt its open reading frame. Further downstream of these elements are the Cγ1 exons containing complementary C_{H}3 mutations for heterotypic association with the mutant IgG1 heavy chain encoded by allele 601.

In a preferred configuration, as in Example 3, the inserted DNA cassette in allele 602 comprises an open reading frame from a gene that provides survival, functional, or selection advantages to the B cells that have successfully assembled an in-frame V_{H} exon from VDJ recombination. The DNA cassette also contains a splice acceptor and a 2A peptide sequence preceding the open reading frame of the advantageous gene. Crucially, the 2A peptide also ensures that the advantageous gene is only expressed in the B lymphocytes that have successfully assembled an in-frame V_{H} exon, and not in the B cells that lack a productive VDJ rearrangement.

When expression of a site-specific DNA recombinase is introduced, the intervening DNA segments flanked by the recognition sequences for the site-specific DNA recombinase is excised from both heavy chain alleles. Subsequently, both heavy chains are now competent for full-length heavy chain expression. As described in Example 1, the two mutant IgG1 heavy chains are mutually dependent on each other for cell surface expression and secretion. Expression of either mutant heavy chain allele alone likely results in B cell death, because cell surface expression of the BCR is also required for the survival of mature B cells (see, e.g., Lam, et al., Cell 90:1073-1083 (1997)).

As in Example 1, mature B cells in the transgenic mice of this example harbor two functional heavy chains per cell with one light chain. The transgenic mice may be used subsequently for the simultaneous immunization with two or more antigens of interest. Hybridomas harboring bispecific antibodies are generated using standard methodologies.

According to a specific aspect, the V (603), D (604), and J (605) genes of both heavy chain alleles (601, 602) comprise human coding sequences with mouse regulatory sequences as described in the co-pending application US Pub. No. 2013/0219535 by Wabl and Killeen. Sequences of the heavy chain "intronic" enhancer (606) as well as all downstream elements, including the heavy chain constant region genes found in a wild-type mouse are described in LOCUS: NG_005838 (1..180,971). On allele 601, the sequence spanning 8,608..168,728 that contain Cδ and C_{H} exons of all downstream isotypes is deleted. Additionally, the Cµ exons of allele 601 (171230..175134 of the locus) are flanked by two directly oriented standard loxP sites. On allele 602, the sequence spanning 8,608..175,134 of the locus containing the C_{H} exons of all isotypes is deleted. An *Itgb7* DNA cassette flanked by two directly oriented loxP sites is inserted at around position 178,000 of the locus. Downstream of the 3' loxP sites on both alleles are sequences of the modified Cγ1 exons (elements 611, 612). Exon 4 of both elements 611 and 612 are mutated to favor heterodimerization with each other over self-dimerization. The mutant Cγ1 cDNA sequences of both heavy chain alleles are specified at [SEQ ID No. 1 and 2],

The preceding merely illustrates the principles of the invention. It will be appreciated that those skilled in the art will be able to devise various arrangements which, although not explicitly described or shown herein, embody the principles of the invention and are included within its spirit and scope. Furthermore, all examples and conditional language recited herein are principally intended to aid the reader in understanding the principles of the invention and the concepts contributed by the inventors to furthering the art, and are to be construed as being without limitation to such specifically recited examples and conditions. Moreover, all statements herein reciting principles, aspects, and embodiments of the invention as well as specific examples thereof, are intended to encompass both structural and functional equivalents thereof. Additionally, it is intended that such equivalents include both currently known equivalents and equivalents developed in the future, i.e., any elements developed that perform the same function, regardless of structure. The scope and spirit of present invention is embodied by the appended claims. In the claims that follow, unless the term "means" is used, none of the features or elements recited therein should be construed as means-plus-function limitations pursuant to applicable patent law

## Claims

1. A genetically modified rodent that produces B lymphocytes each capable of expressing a bispecific antibody, the genetically modified rodent comprising heavy chain alleles comprising unrearranged V, D and J genes, which alleles are engineered to introduce mutations that disfavor homodimeric heavy chain formation but are compatible with heterodimerization between the heavy chains expressed from the alleles, which mutations are any of:
(i) a mutation in an exon that encodes a murine IgG1 C_{H}3 dimerization domain comprising D276K, E233K, and Q234K in a first heavy chain allele and K286D, K269D, and T247D in a second heavy chain allele, wherein amino acid numbering starts at the first codon of C_{H}1; or
(ii) the heavy chains from each allele are fused to a protein or protein domain which is a complementary half of a heterodimerizer pair such as c-Fos and c-Jun, or to a leucine zipper that forms heterodimers.

2. The genetically modified rodent of claim 1, wherein part or all of one or more constant region exons of an immunoglobulin heavy chain gene are in inverted reading frame orientation relative to rearranged V(D)J gene segments in the same immunoglobulin heavy chain gene, wherein the inverted constant region exons are immediately flanked by two oppositely oriented recognition sequences for site-specific DNA recombination.

3. The genetically modified rodent of claim 1 or 2, further comprising a DNA cassette comprising one or more of: a splice acceptor, a ribosomal skip sequence, an internal ribosomal entry site (IRES), an open reading frame, a poly-adenylation signal sequence, and a stop codon inserted downstream of J gene segments of one allele in the heavy chain locus to prevent expression of the constant region (CH) exons downstream of the rearranged V(D)J gene segments on the same chromosome, wherein the DNA cassette is flanked by oppositely oriented site-specific recombinase recognition sequences such that the DNA cassette can be excised or inverted to allow a previously silenced VDJ exon to be spliced to a downstream C_{H} exon for full-length heavy chain expression.

4. The genetically modified rodent of any of claims 1 to 3, comprising an immunoglobulin heavy chain locus comprising mutant Cγ1 shown in SEQ ID NO: 1 and 2.

5. The genetically modified rodent of any of claims 1 to 4, wherein the rodent is a rat or a mouse.

6. A B cell from the genetically modified rodent of any of claims 1 to 5, wherein the B cell is capable of expressing an immunoglobulin comprising two functional V_{H} domains and one functional V_{L} domain, the B cell comprising a heavy chain encoding gene which is engineered in both heavy chain alleles to introduce mutations that disfavor homodimeric heavy chain formation but are compatible with heterodimerization between heavy chains expressed from the alleles, which mutations are any of:
(i) a mutation in an exon that encodes a murine IgG1 C_{H}3 dimerization domain comprising D276K, E233K, and Q234K in a first heavy chain allele and K286D, K269D, and T247D in a second heavy chain allele, wherein amino acid numbering starts at the first codon of C_{H}1; or
(ii) the heavy chains from each allele are fused to a protein or protein domain which is a complementary half of a heterodimerizer pair such as c-Fos and c-Jun, or to a leucine zipper that forms heterodimers.

7. A method of producing a bispecific antibody, the method comprising:
(a) providing the genetically modified rodent of any of claims 1 to 5; and
(b) immunizing the genetically modified rodent with two different antigens simultaneously, or with one antigen followed by a second, different antigen, to generate B cells, each capable of co-expressing, or sequentially expressing, two different antigen receptors and/or a bispecific antigen receptor.

8. A method of generating an immortalized B cell or hybridoma that expresses a bispecific antibody, the method comprising:
(a) providing the genetically modified rodent of any of claims 1 to 5;
(b) immunizing the genetically modified rodent with two different antigens simultaneously, or with one antigen followed by second, different antigen, to generate B cells, each capable of co-expressing, or sequentially expressing, two or more different antigen receptors and/or a bispecific antigen receptor;
(c) isolating B cells specific for both the first and second antigen; and
(d) generating immortalized B cells or a hybridoma using the isolated B cells.

9. The method of claim 7, wherein the bispecific antibody consists of heavy chains only.

10. The method of claim 7 or 8, wherein the bispecific antibody comprises two different light chain proteins and one heavy chain protein.

11. The method of claim 7 or 8, wherein the bispecific antibody comprises one light chain protein and two different heavy chain proteins.

12. A method of cloning immunoglobulin chain genes, the method comprising:
(a) providing the genetically modified rodent of any of claims 1 to 5;
(b) immunizing the genetically modified rodent with two different antigens simultaneously, or with one antigen followed by second, different antigen, to activate B cells, each capable of co-expressing, or sequentially expressing, two different antigen receptors and/or a bispecific antigen receptor;
(c) isolating B cells specific for both the first and second antigen; and
(d) obtaining RNA from the isolated B cells to clone immunoglobulin heavy and light chain genes.

## Patentansprüche

1. Genetisch modifiziertes Nagetier, das B-Lymphozyten produziert, die jeweils in der Lage sind, einen bispezifischen Antikörper zu produzieren, wobei das genetisch modifizierte Nagetier Allele der schweren Kette umfasst, die nicht umgeordnete V-, D- und J-Gene umfasst, wobei die Allele gentechnisch verändert sind, um Mutationen einzuführen, welche die Bildung homodimerer schwerer Ketten behindern, jedoch mit der Heterodimerisierung zwischen schweren Ketten, die von den Allelen exprimiert werden, kompatibel sind, wobei die Mutationen beliebige der folgenden sind:
(i) eine Mutation in einem Exon, das für eine IgG1-C_{H}3-Dimerisierungsdomäne der Maus kodiert, umfassend D276K, E233K und Q234K in einem ersten Allel der schweren Kette und K286D, K269D und T247D in einem zweiten Allel der schweren Kette, wobei die Nummerierung der Aminosäuren an dem ersten Codon von C_{H}1 beginnt; oder
(ii) die schweren Ketten von jedem Allel an ein Protein oder eine Proteindomäne, das/die eine komplementäre Hälfte eines Heterodimerisierer-Paares ist, wie etwa c-Fos und c-Jun, oder an einen Leucin-Zipper fusioniert sind, der Heterodimere bildet.

2. Genetisch modifiziertes Nagetier nach Anspruch 1, wobei ein Teil oder alle von einer oder mehreren Exons der konstanten Region eines Gens der schweren Kette von Immunglobulin in invertierter Leseraster-Orientierung relativ zu umgeordneten V(D)J-Gensegmenten in demselben Gen der schweren Kette von Immunoglobulin vorliegen, wobei die invertierten Exons der konstanten Region unmittelbar von zwei entgegengesetzt orientierten Erkennungssequenzen für die locusspezifische DNA-Rekombination flankiert sind.

3. Genetisch modifiziertes Nagetier nach Anspruch 1 oder 2, ferner umfassend eine DNA-Kassette, die eines oder mehrere aus folgenden umfasst: einen Spleißakzeptor, eine ribosomale Skip-Sequenz, eine interne ribosomale Eintrittsstelle (IRES), ein offenes Leseraster, eine Polyadenylierungs-Signalsequenz und ein Stoppcodon, das den J-Gensegmenten eines Allels in dem Locus der schweren Kette nachgelagert eingefügt ist, um die Expression der Exons der konstanten Region (CH) der umgeordneten V(D)J-Gensegmente auf demselben Chromosom nachgelagert zu verhindern, wobei die DNA-Kassette durch entgegengesetzt orientierte locusspezifische Rekombinase-Erkennungssequenzen flankiert ist, so dass die DNA-Kassette ausgeschnitten oder invertiert werden kann, um zu ermöglichen, dass ein zuvor abgeschaltetes VDJ-Exon an ein nachgelagertes CH-Exon für die Expression der schweren Kette in voller Länge gespleißt wird.

4. Genetisch modifiziertes Nagetier nach einem der Ansprüche 1 bis 3, umfassend einen Locus der schweren Kette von Immunoglobulin, der das in SEQ ID NO: 1 und 2 gezeigte mutierte Cγ1 umfasst.

5. Genetisch modifiziertes Nagetier nach einem der Ansprüche 1 bis 4, wobei das Nagetier eine Ratte oder eine Maus ist.

6. B-Zelle von dem genetisch modifizierten Nagetier nach einem der Ansprüche 1 bis 5, wobei die B-Zelle in der Lage ist, ein Immunglobulin zu exprimieren, das zwei funktionale VH-Domänen und eine funktionale VL-Domäne umfasst, wobei die B-Zelle ein für die schwere Kette kodierendes Gen umfasst, das in beiden Allelen der schweren Kette gentechnisch verändert ist, um Mutationen einzuführen, welche die Bildung homodimerer schwerer Ketten behindern, jedoch mit der Heterodimerisierung zwischen schweren Ketten, die von den Allelen exprimiert werden, kompatibel sind, wobei die Mutationen beliebige der folgenden sind:
(i) eine Mutation in einem Exon, das für eine IgG1-C_{H}3-Dimerisierungsdomäne der Maus kodiert, umfassend D276K, E233K und Q234K in einem ersten Allel der schweren Kette und K286D, K269D und T247D in einem zweiten Allel der schweren Kette, wobei die Nummerierung der Aminosäuren an dem ersten Codon von C_{H}1 beginnt; oder
(ii) die schweren Ketten von jedem Allel an ein Protein oder eine Proteindomäne, das/die eine komplementäre Hälfte eines Heterodimerisierer-Paares ist, wie etwa c-Fos und c-Jun, oder an einen Leucin-Zipper fusioniert sind, der Heterodimere bildet.

7. Verfahren zur Herstellung eines bispezifischen Antikörpers, wobei das Verfahren umfasst:
(a) Bereitstellen des genetisch modifizierten Nagetiers nach einem der Ansprüche 1 bis 5; und
(b) Immunisieren des genetisch modifizierten Nagetiers mit zwei unterschiedlichen Antigenen gleichzeitig, oder mit einem Antigen gefolgt von einem zweiten unterschiedlichen Antigen, um B-Zellen zu erzeugen, die jeweils in der Lage sind, zwei unterschiedliche Antigen-Rezeptoren und/oder einen bispezifischen Antigen-Rezeptor gleichzeitig oder sequenziell zu exprimieren.

8. Verfahren zur Herstellung einer immortalisierten B-Zelle oder eines immortalisierten Hybridoms, die/das einen bispezifischen Antikörper exprimiert, wobei das Verfahren umfasst:
(a) Bereitstellen des genetisch modifizierten Nagetiers nach einem der Ansprüche 1 bis 5;
(b) Immunisieren des genetisch modifizierten Nagetiers mit zwei unterschiedlichen Antigenen gleichzeitig, oder mit einem Antigen gefolgt von einem zweiten unterschiedlichen Antigen, um B-Zellen zu erzeugen, die jeweils in der Lage sind, zwei oder mehr unterschiedliche Antigen-Rezeptoren und/oder einen bispezifischen Antigen-Rezeptor gleichzeitig oder sequenziell zu exprimieren;
(c) Isolieren der B-Zellen, die sowohl für das erste als auch das zweite Antigen spezifisch sind; und
(d) Erzeugen von immortalisierten B-Zellen oder eines immortalisierten Hybridoms unter Verwendung der isolierten B-Zellen.

9. Verfahren nach Anspruch 7, wobei der bispezifische Antikörper nur aus schweren Ketten besteht.

10. Verfahren nach Anspruch 7 oder 8, wobei der bispezifische Antikörper zwei unterschiedliche Leichtketten-Proteine und ein Schwerketten-Protein umfasst.

11. Verfahren nach Anspruch 7 oder 8, wobei der bispezifische Antikörper ein Leichtketten-Protein und zwei unterschiedliche Schwerketten-Proteine umfasst.

12. Verfahren zur Klonierung von Immunglobulin-Ketten-Genen, wobei das Verfahren umfasst:
(a) Bereitstellen des genetisch modifizierten Nagetiers nach einem der Ansprüche 1 bis 5;
(b) Immunisieren des genetisch modifizierten Nagetiers mit zwei unterschiedlichen Antigenen gleichzeitig, oder mit einem Antigen gefolgt von einem zweiten unterschiedlichen Antigen, um B-Zellen zu aktivieren, die jeweils in der Lage sind, zwei unterschiedliche Antigen-Rezeptoren und/oder einen bispezifischen Antigen-Rezeptor gleichzeitig oder sequenziell zu exprimieren;
(c) Isolieren der B-Zellen, die sowohl für das erste als auch das zweite Antigen spezifisch sind; und
(d) Gewinnen von RNA aus den isolierten B-Zellen, um Gene der schweren Kette und der leichten Kette von Immunglobulin zu klonen.

## Revendications

1. Rongeur génétiquement modifié qui produit des lymphocytes B capables chacun d'exprimer un anticorps bispécifique, le rongeur génétiquement modifié comprenant des allèles de chaînes lourdes comprenant des gènes V, D et J non réarrangés, lesquels allèles sont modifiés pour introduire des mutations qui défavorisent la formation de chaînes lourdes homodimères mais sont compatibles avec l'hétérodimérisation entre les chaînes lourdes exprimées à partir des allèles, lesquelles mutations sont l'une quelconque parmi :
(i) une mutation dans un exon qui code un domaine de dimérisation de C_{H}3 d'IgG1 murine comprenant D276K, E233K et Q234K dans un premier allèle de chaîne lourde et K286D, K269D et T247D dans un second allèle de chaîne lourde, dans laquelle la numérotation des acides aminés commence au niveau du premier codon de C_{H}1 ; ou
(ii) les chaînes lourdes provenant de chaque allèle sont fusionnées à une protéine ou à un domaine de protéine qui est une moitié complémentaire d'une paire d'hétérodimériseurs telle que c-Fos et c-Jun, ou à une glissière à leucine qui forme des hétérodimères.

2. Rongeur génétiquement modifié selon la revendication 1, dans lequel une partie ou la totalité d'un ou plusieurs exons de région constante d'un gène de chaîne lourde d'immunoglobuline se trouvent dans une orientation de cadre de lecture inversée par rapport aux segments de gène V(D)J réarrangés dans le même gène de chaîne lourde d'immunoglobuline, dans lequel les exons de région constante inversés sont immédiatement flanqués de deux séquences de reconnaissance orientées de manière opposée pour une recombinaison d'ADN sito-spécifique.

3. Rongeur génétiquement modifié selon la revendication 1 ou 2, comprenant en outre une cassette d'ADN comprenant un ou plusieurs éléments parmi : un accepteur d'épissage, une séquence de saut ribosomique, un site d'entrée ribosomique interne (IRES), un cadre de lecture ouvert, une séquence signal de polyadénylation et un codon d'arrêt inséré en aval des segments de gène J d'un allèle dans le locus de chaîne lourde pour empêcher l'expression des exons de région constante (CH) en aval des segments de gène v(D)J réarrangés sur le même chromosome, dans lequel la cassette d'ADN est flanquée de séquences de reconnaissance de recombinase sito-spécifiques orientées de manière opposée de sorte que la cassette d'ADN puisse être excisée ou inversée pour permettre l'épissage d'un exon de VDJ préalablement rendu silencieux jusqu'à un exon de C_{H} en aval pour l'expression de chaînes lourdes pleine longueur.

4. Rongeur génétiquement modifié selon l'une quelconque des revendications 1 à 3, comprenant un locus de chaîne lourde d'immunoglobuline comprenant le mutant Cγ1 présenté dans les SEQ ID N°: 1 et 2.

5. Rongeur génétiquement modifié selon l'une quelconque des revendications 1 à 4, dans lequel le rongeur est un rat ou une souris.

6. Lymphocyte B provenant d'un rongeur génétiquement modifié selon l'une quelconque des revendications 1 à 5, dans lequel le lymphocyte B est capable d'exprimer une immunoglobuline comprenant deux domaines V_{H} fonctionnels et un domaine V_{L} fonctionnel, le lymphocyte B comprenant un gène de codage de chaîne lourde qui est modifié dans les deux allèles de chaînes lourdes pour introduire des mutations qui défavorisent la formation de chaînes lourdes homodimères mais sont compatibles avec l'hétérodimérisation entre des chaînes lourdes exprimées à partir des allèles, lesquelles mutations sont l'une quelconque parmi :
(i) une mutation dans un exon qui code un domaine de dimérisation de C_{H}3 d'IgG1 murine comprenant D276K, E233K et Q234K dans un premier allèle de chaîne lourde et K286D, K269D et T247D dans un second allèle de chaîne lourde, dans laquelle la numérotation des acides aminés commence au niveau du premier codon de C_{H}1 ; ou
(ii) les chaînes lourdes provenant de chaque allèle sont fusionnées à une protéine ou à un domaine de protéine qui est une moitié complémentaire d'une paire d'hétérodimériseurs telle que c-Fos et c-Jun, ou à une glissière à leucine qui forme des hétérodimères.

7. Procédé de production d'un anticorps bispécifique, le procédé comprenant :
(a) la fourniture du rongeur génétiquement modifié selon l'une quelconque des revendications 1 à 5 ; et
(b) l'immunisation du rongeur génétiquement modifié avec deux antigènes différents en même temps, ou avec un antigène suivi d'un second antigène différent pour générer des lymphocytes B, capables chacun de co-exprimer, ou d'exprimer de manière séquentielle, deux récepteurs d'antigène différents et/ou un récepteur d'antigène bispécifique.

8. Procédé de génération d'un lymphocyte B immortalisé ou d'un hybridome qui exprime un anticorps bispécifique, le procédé comprenant :
(a) la fourniture du rongeur génétiquement modifié selon l'une quelconque des revendications 1 à 5 ;
(b) l'immunisation du rongeur génétiquement modifié avec deux antigènes différents en même temps, ou avec un antigène suivi d'un second antigène différent pour générer des lymphocytes B, capables chacun de co-exprimer, ou d'exprimer de manière séquentielle, deux récepteurs d'antigène différents ou plus et/ou un récepteur d'antigène bispécifique ;
(c) l'isolement des lymphocytes B spécifiques à aussi bien le premier que le second antigène ; et
(d) la génération de lymphocytes B immortalisés ou d'un hybridome à l'aide des lymphocytes B isolés.

9. Procédé selon la revendication 7, dans lequel l'anticorps bispécifique est constitué de chaînes lourdes uniquement.

10. Procédé selon la revendication 7 ou 8, dans lequel l'anticorps bispécifique comprend deux protéines de chaîne légère différentes et une protéine de chaîne lourde.

11. Procédé selon la revendication 7 ou 8, dans lequel l'antigène bispécifique comprend une protéine de chaîne légère et deux protéines de chaîne lourde différentes.

12. Procédé de clonage de gènes de chaînes d'immunoglobulines, le procédé comprenant :
(a) la fourniture du rongeur génétiquement modifié selon l'une quelconque des revendications 1 à 5 ;
(b) l'immunisation du rongeur génétiquement modifié avec deux antigènes différents en même temps, ou avec un antigène suivi d'un second antigène différent pour générer des lymphocytes B, capables chacun de co-exprimer, ou d'exprimer de manière séquentielle, deux récepteurs d'antigène différents et/ou un récepteur d'antigène bispécifique ;
(c) l'isolement des lymphocytes B spécifiques à aussi bien le premier que le second antigène ; et
(d) l'obtention d'ARN provenant des lymphocytes B isolés pour cloner les gènes de chaînes légères et lourdes d'immunoglobulines.
